# EUROPÄISCHE PATENTANMELDUNG

(11) **EP 4 813 362 A1**
(43) Veröffentlichungstag der Anmeldung: **30.09.2026**
(21) Anmeldenummer: 25167207.7
(22) Anmeldetag: 28.03.2025
(51) Int. Cl.: A61F 13/00, A61F 13/01, A61F 13/0206, A61L 15/46, A61L 26/00

(54) **ABSORBIERENDE WUNDAUFLAGE MIT WIRKSAMKEIT GEGENÜBER BIOFILMEN**

(71) Anmelder: Paul Hartmann AG, 89522 Heidenheim (DE)
(72) Erfinder: Kettel, Markus, 89522 Heidenheim (DE); Merckel, Fabien, 67100 Strasbourg (FR); Nosworthy, Jonathan, 89231 Neu-Ulm (DE); Law, Stephen, Liverpool, L78XZ (GB); Percival, Steven, Liverpool, L78XZ (GB); Chen, Rui, Liverpool, L78XZ (GB)
(74) Vertreter: Paul Hartmann AG Patents & Licensing

(57) **Zusammenfassung**

Die vorliegende Erfindung betrifft eine stark saugfähige Wundauflage mit antimikrobieller Zusammensetzung. Die Wundauflage umfasst ein Kissen, das einen absorbierenden Kern enthält. Die antimikrobielle Zusammensetzung umfasst die Inhaltsstoffe Silber, Zink und EDTA und entfaltet ein breites Wirkungsspektrum sowohl gegen schädliche Mikroorganismen als auch die von diesen Erregern gebildeten Biofilme.

## Beschreibung

### Technisches Gebiet der Erfindung

Die vorliegende Erfindung betrifft die Wundbehandlung, insbesondere die Behandlung von Wunden, die infiziert sind oder bei denen die Gefahr einer Infektion besteht. Hierzu gehören chronische und/oder exsudierende Wunden als auch solche Wunden, bei denen eine vorliegende Infektion bereits zur Bildung eines Biofilms in der Wunde geführt hat.

### Hintergrund der Erfindung

Chronische Wunden stellen in der modernen Medizin nach wie vor ein Problem dar. Gerade bei älteren Menschen und Risikopatienten wie beispielsweise Diabetikern besteht ein erhöhtes Risiko, dass Verletzungen nicht oder nicht vollständig abheilen. Der Wundheilungsprozess ist in einem solchen Fall aus unterschiedlichen Gründen gestört und es besteht ein fortwährender Defekt der Hautbarriere. Dies geht zum einen mit einer Reduktion der Lebensqualität für die Betroffenen einher. Zum anderen erhöht sich mit fortwährender Dauer der Gewebeexposition zunehmend das Risiko einer Infektion. Tritt eine solche Infektion ein, verschlechtert sich die Prognose weiter. Da die körpereigenen Stoffwechselprozesse im Bereich der chronischen Wunde beeinträchtigt sind und eine reguläre Immunantwort nicht oder nicht vollständig erfolgt, kann es in der Folge zu einer Vermehrung der Erreger an der Infektionsstelle kommen. Im weiteren Verlauf bildet sich häufig ein Biofilm, bei dem bakterielle Erreger sich zu einer Lebensgemeinschaft vereinen, die erhöhte Resistenz gegenüber Bioziden und Antibiotika zeigt und daher die weitere Behandlung außerordentlich erschwert. Dieses Stadium erhöht die Wahrscheinlichkeit für weitere Komplikationen wie beispielsweise Nekrosenbildung oder Sepsis.

Aus dem Stand der Technik sind Wundauflagen mit antibiotischer Wirksamkeit bekannt. Diese sind jedoch entweder unzureichend wirksam gegenüber Biofilmen oder sind aufgrund ungenügender Absorptionseigenschaften ungeeignet, um bei nässenden Wunden eingesetzt zu werden (unzureichendes Exsudatmanagement). Allerdings neigen gerade die erwähnten chronischen Wunden dazu große Mengen an Wundexsudat abzugeben. Nicht abgeführtes Wundexsudat kann die Wundränder aufweichen (Mazeration) und darüber hinaus dazu führen, dass Wachstumsfaktoren sowie Fibroblasten nicht im notwendigen Ausmaß innerhalb der Wunde zur Verfügung stehen. In der Folge kommt es zur Stagnation der Heilungsreaktion.

Die EP 1 755 569 B9 beschreibt eine Wundauflage mit Salbe, die zusätzlich ein antibakterielles Metall wie beispielsweise Silber enthält. Allerdings ist diese Wundauflage weder für die Behandlung nässender Wunden geeignet, noch ist sie effektiv bei der Versorgung von mit Biofilm durchsetzten Wunden.

Folglich besteht ein Bedarf nach einem Mittel zur Wundabdeckung, das ausreichende antimikrobielle Wirksamkeit auch gegenüber Biofilmen zeigt und in der Lage ist große Mengen an Wundexsudat im Rahmen eines effektiven Exsudatmanagements zu absorbieren, um einer Aufweichung des Gewebes und der Wundränder vorzubeugen. Um dem klinischen Alltag gerecht zu werden, sollte das Mittel der Wahl zudem unmittelbar einsetzbar sein, bei längerer Lagerung über eine ausreichende Stabilität verfügen und beständig gegenüber möglichen Temperaturschwankungen sein.

### Zusammenfassung der Erfindung

Die zuvor genannte Aufgabe wird gelöst durch eine Wundauflage umfassend ein Kissen, wobei das Kissen eine erste wundzugewandte Vliesschicht und eine zweite wundabgewandte Vliesschicht aufweist, wobei das Material der ersten Vliesschicht flüssigkeitsdurchlässig ist und sich das Material der zweiten Vliesschicht von dem Material der ersten Vliesschicht unterscheidet, wobei das Kissen weiterhin einen absorbierenden Kern umfassend eine superabsorbierende Substanz enthält und die erste Vliesschicht und die zweite Vliesschicht den absorbierenden Kern einschließen und dabei gemeinsam einen den absorbierenden Kern umgebenden, durchgehenden Randbereich ausbilden, in dem die erste Vliesschicht und die zweite Vliesschicht miteinander verbunden sind,
dadurch gekennzeichnet, dass die erste Vliesschicht eine antimikrobielle Zusammensetzung aufweist, die die Inhaltsstoffe
a) ionisches Silber oder Silbernitrat,
b) ionisches Zink, Zinksulfat oder Zinknitrat, und
c) EDTA oder Tetra-Natrium-EDTA
beinhaltet.

Die erfindungsgemäße Wundauflage ist aufgrund ihrer antimikrobiellen Inhaltsstoffe gegen humanpathogene Erreger und deren Biofilme wirksam. Darüber hinaus ist die erfindungsgemäße Wundauflage in der Lage große Mengen an Wundexsudat zu absorbieren und das aufgenommene Exsudat zu speichern. Die Wundauflage kann dabei für die Behandlung unterschiedlicher Wundarten und Einsatzbedingungen spezifisch zusammengestellt werden, um die bestmögliche Versorgung zu gewährleisten.

Die erfindungsgemäße Wundauflage kann darüber hinaus auch dann zum Einsatz kommen, wenn sich in der zu behandelnden Wunde antibiotikaresistente Bakterien befinden. Denn während Antibiotika üblicherweise organische Verbindungen sind, die von resistenten Bakterien abgebaut, gespalten oder anderweitig deaktiviert werden können, ist ein derartiges Auftreten von Resistenzen im Hinblick auf die antimikrobiellen Inhaltsstoffe der vorliegenden Erfindung nicht beobachtet worden.

Weiterhin kann die erfindungsgemäße Wundauflage wundheilungsfördernde Eigenschaften aufweisen. Dies wird erreicht, durch die Verwendung von Zink, was die Epithelbildung stimuliert, cytoprotektive Eigenschaften hat und eine entzündungshemmende Wirkung entfaltet.

Die im Rahmen der Wundbehandlung freigesetzten Kationen von Zink und Silber können mittels der ihnen anhaftenden Hydrathülle der Wunde Feuchtigkeit zuführen. Ein kontrolliertfeuchtes Wundmilieu fördert den Heilungsprozess.

Bei der Wundauflage kann es sich um einen absorbierenden bzw. superabsorbierenden Verband, einen Inselverband oder eine Kompresse handeln. Der erste Vliesstoff kann innerhalb der Wundauflage als Wundkontaktschicht vorliegen.

Nachfolgend wird erläutert, wie die Wundauflage strukturell und chemisch beschaffen sein kann, um in der Praxis den größtmöglichen Nutzen zu erbringen.

### Detaillierte Beschreibung der Erfindung

Der Begriff "medizinisch akzeptables Material" im Sinne der Erfindung ist eine ungiftige, fusselfreie und stabile Substanz (z.B. ein Substrat), die sich unter Normalbedingungen weder in polaren noch in unpolaren Verbindungen löst und weder von den Absonderungen tierischer noch bakterieller Zellen in nennenswertem Ausmaß abgebaut oder verflüssigt werden kann.

Der Begriff "Vliesstoff" meint eine Schicht von miteinander zusammenhängenden Fasern, die nicht gewebt sind und in der Regel nicht nach einem sich wiederholendem Muster angeordnet sind.

Der Ausdruck "Kolonie bildende Einheit" (CFU) meint eine einzelne teilungsfähige Zelle eines Einzellers, insbesondere eines humanpathogenen Einzellers oder Bakteriums.

"Beschichtet" meint, dass eine Oberfläche eines Festkörpers (z.B. eines Vliesstoffes) mittels einer Substanz, die sich von der Struktur des Festkörpers unterscheidet, zumindest teilweise bedeckt bzw. überlagert wird. Insbesondere kann die Beschichtung faserlos und / oder hydrophil sein.

Der Ausdruck "konfiguriert zum Auflegen auf eine Wunde" meint zum Auflegen auf eine Wunde zu einem therapeutischen Zweck im Sinne einer Wundbehandlung vorgesehen und geeignet.

Der Ausdruck "antimikrobielle Inhaltsstoffe" meint a) Silber - insbesondere ionisches Silber - sowie Silbersalze, b) Zink - insbesondere ionisches Zink - sowie Zinksalze und c) EDTA sowie EDTA-Salze. Die Inhaltsstoffe a) bis c) ergeben dabei gemeinsam die erfindungsgemäße antimikrobielle Zusammensetzung.

Mit "t-EDTA" ist die Verbindung Tetra-Natrium-Ethylendiamintetraessigsäure gemeint.

Der Ausdruck "antimikrobiell" meint im weitesten Sinne, dass ein Inhaltsstoff, Inhaltsstoffgemisch oder ein damit behandelter Artikel (z.B. eine Wundauflage) in der Lage ist, die Vermehrung von Mikroorganismen zu hemmen oder aufzuhalten oder die Anzahl vitaler Mikroorganismen zu reduzieren. Im engeren Sinne ist gemeint, dass ein solcher Artikel in der Lage ist, in einem Test nach ISO 20743:2021 die Anzahl der CFU des *Pseudomonas aeruginosa* Stammes mit der Hinterlegungsnummer ATTC 27853 und / oder des *Staphylococcus aureus* Stammes mit der Hinterlegungsnummer ATTC 25923 um mindestens drei Logarithmusstufen, bevorzugt um mindestens vier Logarithmusstufen, besonders bevorzugt um mindestens fünf Logarithmusstufen und ganz besonders bevorzugt um mindestens sechs Logarithmusstufen im Vergleich zu einem baugleichen Artikel ohne Wirkstoff zu reduzieren. Darüber hinaus schließt der Ausdruck "antimikrobiell" den Ausdruck "antibakteriell" mit ein.

Der Begriff "Biofilm" meint einen dünnen, flächig ausgebreiteten Schleimfilm, in dem Populationen von Mikroorganismen vorliegen. Der Schleimfilm wird von den Mikroorganismen gebildet und ist eine die Mikroorganismen einschließende Matrix aus extrazellulärem polymerem Material. Typischerweise handelt es sich bei den Populationen von Mikroorganismen um Mischpopulationen. Biofilme bilden sich auf Oberflächen aus, zu denen auch Wundgewebe gehören kann. Die im Biofilm organisierten Mikroorganismen zeigen eine erhöhte Widerstandskraft gegen herkömmliche Antibiotika, Desinfektionsmittel und gegenüber dem Immunsystem höher entwickelter Lebewesen.

Der Begriff "Biofilm reduzierend" umfasst sowohl die Abtötung von Bakterien im Biofilm (desinfizierende Eigenschaften) als auch das Aufbrechen von chemischen Bindungen und den Entzug chemischer Bindungspartner aus der Biofilm bildenden extrazellulären Matrix. Letzteres setzt die Abwehrkraft der zuvor im Biofilm organisierten Mikroorganismen gegenüber Einflüssen wie Desinfektionsmitteln, Antibiotika, dem Immunsystem oder Umwelteinflüssen allgemein herab. Als Biofilm reduzierend werden im Rahmen der vorliegenden Erfindung nur derartige Substanzen verstanden, die geeignet sind, auf offene Wunden aufgebracht zu werden, ohne eine nennenswerte Schadwirkung am zu behandelnden Individuum zu entfalten.

"Beschichtet" meint, dass eine Oberfläche eines Festkörpers mittels einer Substanz, die sich von der Struktur des Festkörpers unterscheidet, zumindest teilweise bedeckt bzw. überlagert wird.

Der Ausdruck "wundzugewandt" bedeutet, dass ein Objekt oder Material in Benutzung zur Wunde oder Haut ausgerichtet ist, aber diese nicht zwangsläufig berühren muss. Ein anderer Begriff für "wundzugewandt" ist "proximal".

Der Ausdruck "distal" bedeutet, dass ein Element im Einsatz von der Wunde oder Haut weg zeigt bzw. wundabgewandt ist.

Die vorliegende Erfindung betrifft eine Wundauflage. Diese Wundauflage kann allein verwendet werden oder nach dem Anbringen auf eine Wunde bei Bedarf durch einen Sekundärverband, Klebefolie, Klebestreifen oder mithilfe anderer Fixiermittel am Ort der Wunde befestigt werden. Gegenstand der Erfindung sind auch Wundauflagen, die mit optionalen Klebeflächen ausgestattet sind und ohne Hilfsmittel im Bereich der Wunde angeklebt werden können. Eine mögliche Ausgestaltung einer solchen Wundauflage ist der sogenannte Inselverband mit umlaufendem Kleberand. Dieser Kleberand kann mittels hautkompatibler Klebstoffe bereitgestellt werden, wobei Acrylkleber und Silikonkleber zu den gebräuchlichsten Mitteln gehören. Diese haben gleichzeitig den Vorteil restlos ablösbar zu sein. Eine andere mögliche Ausgestaltung der erfindungsgemäßen Wundauflage ist ein sogenanntes Sandwichdressing. Hierbei wird das Kissen der Wundauflage allseitig sowohl von einer wundabgewandt liegenden Deckschicht als auch von einer wundzugewandt liegenden Wundkontaktschicht überragt, so dass sie einen umlaufenden Rand um das Kissen bilden und wobei die Deckschicht und die Wundkontaktschicht zumindest teilweise im Randbereich miteinander verbunden (z.B. verklebt) sind. Die Außenseite der Wundkontaktfläche eines solchen Sandwichdressings kann ganz oder teilweise mit einem Klebstoff beschichtet sein, um das Sandwichdressing an der Haut und / oder Wunde zu befestigen. Bei einer vollflächigen Klebebeschichtung wird der Einsatz von atraumatischen, wundkompatiblen Klebstoffen wie z.B. Silikonkleber empfohlen.

Die erfindungsgemäße Wundauflage eignet sich für akute (z.B. blutende) Wunden als auch für chronische Wunden. Insbesondere die bei chronischen Wunden häufig vorkommenden nässenden (exsudierenden) und / oder infizierten Wundformen können hervorragend vorsorgt werden. Beispiele für mögliche exsudierende Wunden sind Dekubitus, Ulcus Cruris sowie exulzerierende Tumore. In der Praxis treten häufig Mischformen der aufgeführten Wundvarianten auf, in welchen die Erreger bereits in Form eines Biofilms vorliegen. In solchen Fällen zeigen sich die vorteilhaften Eigenschaften der erfindungsgemäßen Wundauflage besonders deutlich.

Das in der Wundauflage enthaltene Kissen umfasst die folgenden Bestandteile:
- Eine erste Vliesschicht, die während des Einsatzes wundzugewandt ausgerichtet ist. Diese erste Vliesschicht kann als Wundkontaktschicht fungieren. Für gewöhnlich ist diese Schicht hydrophil oder hat hydrophile Anteile, um die Absorption von Flüssigkeiten in das Kissen zu erleichtern. Allerdings kann diese Schicht durchaus auch hydrophobe Faserarten enthalten. Zudem können auch hydrophobe Faserarten vorliegen, welche zuvor einer Hydrophilierung unterzogen wurden.
- Eine zweite Vliesschicht, die aus einem anderen Material oder Materialgemisch als die erste Vliesschicht besteht und während des Einsatzes wundabgewandt ausgerichtet ist. Für gewöhnlich ist diese Schicht hydrophob. Dies bietet zum einen den Vorteil, dass Flüssigkeit, die in das Kissen aufgenommen wurde, nicht durch diese Schicht nach außen dringt und zum anderen den Vorteil, dass von Außen auf das Kissen gelangte Flüssigkeit (z.B. im Rahmen der Körperpflege) von der Kissenoberfläche abgewiesen wird.
- Einen durchgehenden Randbereich, in dem die erste Vliesschicht und die zweite Vliesschicht miteinander verbunden sind.
- Einen absorbierenden Kern, der vom Randbereich umgeben ist und somit von der ersten sowie der zweiten Vliesschicht eingeschlossen wird. Der absorbierende Kern umfasst eine superabsorbierende Substanz.
- Eine antimikrobielle Zusammensetzung, die die Inhaltsstoffe
   a) ionisches Silber oder Silbernitrat,
   b) ionisches Zink, Zinksulfat oder Zinknitrat, und
   c) EDTA oder Tetra-Natrium-EDTA
   beinhaltet. Vorzugsweise sind diese Inhaltsstoffe dazu geeignet einen Metallkomplex auszubilden. Besonders bevorzugt handelt es sich bei diesem Metallkomplex um Ag₂Zn(EDTA).

Optional kann eine dritte Vliesschicht vorhanden sein, welche die erste Vliesschicht wundseitig überlagert.

Sowohl die erste als auch die zweite Vliesschicht des Kissens und eine optionale dritte Vliesschicht können jeweils Kunstfasern enthalten. Die Anwesenheit von Kunstfasern schließt dabei die Beimischung anderer Faserarten (z.B. natürliche Fasern oder Regeneratfasern) nicht aus. Geeignete Kunstfaserarten sind: Acrylnitril-Butadien-Styrol (ABS), Polyamid (PA), Polylactid (PLA), Polymethylmethacrylat (PMMA), Polycarbonat (PC), Polyethylenterephthalat (PET), Polyethylen (PE), Polypropylen (PP), Polystyrol (PS), Polyetheretherketon (PEEK) oder Polyvinylchlorid (PVC). Vorzugsweise bestehen die in der ersten Vliesschicht und der zweiten Vliesschicht enthaltenen Kunststofffasern aus Polyamid (PA), Polyethylenterephthalat (PET), Polyethylen (PE), Polypropylen (PP) oder Polyvinylchlorid (PVC).

Mögliche Mischungen von Faserarten sind eine Mischung aus Polyamidfasern und Viskosefasern, eine Mischung aus Polypropylenfasern und Viskosefasern, eine Mischung von Polyethylenfasern und Viskosefasern, eine Mischung aus Polyesterfasern mit Baumwollfasern oder eine Mischung aus Polyesterfasern mit Viskosefasern.

Das Material der ersten und / oder zweiten Vliesschicht sowie einer bei Bedarf optional vorhandenen dritten Vliesschicht kann thermisch verfestigt sein. Hierdurch wird die Wahrscheinlichkeit, dass sich aus dem Textilverbund Fasern lösen und in die Wunde gelangen können, stark verringert. In diesem Sinne kann es sich bei dem erfindungsgemäßen Vliesstoff um einen luftgelegten Vliesstoff, einen thermisch verfestigten Vliesstoff, einen mechanisch verfestigten Vliesstoff, einen Stapelfaservliesstoff, einen Schmelzblasvliesstoff, einen Spinnvliesstoff, einen Nassvliesstoff, einen Wirrlagevliesstoff (isotroper Vliesstoff) oder einen anisotropen Vliesstoff (orientierter Vliesstoff) handeln.

Der Vliesstoff oder seine Fasern können die folgenden Materialien oder Materialgemische enthalten oder daraus bestehen: Kunstfasern, Polyolefin basierte Fasern, Polyethylen, Polyetheretherketon (PEEK), Polyvinylchlorid (PVC), Polymethylmethacrylat (PMMA), Polykarbonat (PC), Polyester, Polyethylenterephthalat (PET), Polypropylen (PP), Polystyren (PS), Polyamid, Acrylnitril-Butadien-Styrol (ABS), Polylactide (PLA), Viskose, Zellulose basierte Fasern oder Mischungen aus zwei oder mehr der zuvor genannten Materialien. Bei Verwendung von Polyamid als Fasermaterial kann das Polyamid in Form von Nylon vorliegen.

Synthetische Fasern wie beispielsweise Polyamid und Polyester haben den Vorteil, dass sie beständig sind gegen Zersetzung durch Mikroorganismen. Weiterhin haben viele dieser Fasern wie beispielsweise Polypropylen oder Polyamid hervorragende thermoplastische Eigenschaften, was das Verarbeiten und Zusammenfügen von Schichten (z.B. mittels Schweißen) vereinfacht. Das Zusammenfügen von Schichten ist auch dann möglich, wenn die thermoplastischen Fasern in einem Gemisch mit nicht thermoplastischen Fasern (z.B. Viskose oder Baumwolle) vorliegen.

Der Vliesstoff enthält Fasern oder besteht ausschließlich aus Fasern. Bei den Fasern kann es sich um Fasern der oben genannten Materialien handeln, also zum Beispiel um Polyamidfasern, insbesondere Nylonfasern, Polyesterfasern, Polypropylenfasern, Baumwollfasern, Viskosefasern usw. Darüber hinaus können auch Mischfasern verwendet werden, welche eine Kombination aus zwei oder mehr der aufgeführten Materialien in einer Faser vereinen.

Möglich ist, dass die erste und / oder zweite Vliesschicht sowie eine optional vorhandene dritte Vliesschicht ausschließlich solche Fasern enthalten, die synthetischen Ursprungs sind und / oder solche Fasern, die zu den Kunstfasern gezählt werden. Darüber hinaus können insbesondere die erste Vliesschicht sowie die optional vorhandene dritte Vliesschicht auch Regeneratfasern wie zum Beispiel Viskose enthalten.

Weiterhin ist auch möglich, dass der erfindungsgemäße Vliesstoff ausschließlich solche Fasern enthält, die natürlichen Ursprungs sind und / oder biologisch abbaubar sind. Bei der biologischen Abbaubarkeit kann es sich um eine Abbaubarkeit im Sinne der Norm EN ISO 14851:2019 handeln. Fasern natürlichen Ursprungs sind beispielsweise Baumwollfasern. Fasern, die im Kontext der vorliegenden Erfindung als biologisch abbaubar gelten, sind beispielsweise Baumwollfasern und Viskosefasern. Im Gegensatz dazu sind Kunstfasern nicht biologisch abbaubar.

Unabhängig davon, ob die erste Vliesschicht aus einer einzigen Lage oder mehreren Einzelvliesschichten aufgebaut ist, kann sie insgesamt 50 bis 70 Gew.-% Kunstfasern und 30 bis 50 Gew.-% Viskosefasern enthalten. Das gleich gilt für eine optional vorhandene dritte Vliesschicht. Hierdurch wird sichergestellt, dass die erste Vliesschicht sowie die optional vorhandene dritte Vliesschicht Wundflüssigkeit in den Kern leitet (Dochteffekt). Eine Zusammensetzung der ersten und dritten Vliesschicht, die sich als besonders vorteilhaft erwiesen hat, sind 60 bis 70 Gew.-% Polypropylenfasern und 30 bis 40 Gew.-% Viskosefasern. Die erste und die dritte Vliesschicht können jeweils für sich ein Flächengewicht von 30 bis 60 g/m² haben, bevorzugt 40 bis 50 g/m². Die zweite Vliesschicht kann ein Flächengewicht von 20 bis 50 g/m² haben, bevorzugt 20 bis 30 g/m² und insbesondere 23 bis 27 g/m². Im Allgemeinen gilt, dass ein niedriges Flächengewicht die Atmungsaktivität und Anschmiegsamkeit eines Textilmaterials begünstigt, wohingegen hohe Flächengewichte das Durchleiten von Flüssigkeiten verlangsamen können.

Weiterhin kann die lineare Dichte des Vliesstoffes oder dessen Schichten 60 bis 100 dtex, bevorzugt 70 bis 90 dtex gemessen mittels DIN EN ISO 2060 betragen. Die Zähigkeit des Vliesstoffes kann 30 bis 50 cN/tex, bevorzugt 35 bis 40 cN/tex, gemessen mittels DIN EN ISO 2062 betragen.

Der Vliesstoff oder dessen Schichten können eine Dehnbarkeit nach DIN EN ISO 1798:2008-04 von mindestens 25%, bevorzugt von mindestens 35% haben. Dehnbarkeit ist dahingehend zu verstehen, dass es bei einer entsprechenden Verlängerung des Materials unter Zugkraft nicht zu einem Belastungsbruch oder zu einem Reißen von Fasern kommt. Bevorzugt ist die Dehnbarkeit eine reversible Verformung (Elastizität), so dass das Material nach Aufhebung der Zugkraft im Wesentlichen seine ursprüngliche Länge einnimmt. Eine reversible Dehnbarkeit liegt auch dann vor, wenn das Material nach Aufhebung der Zugkraft eine Länge einnimmt, die maximal 105% der Ausgangslänge entspricht. Die Länge hat dabei dieselbe räumliche Ausrichtung wie die Zugkraft, ist also in Richtung der Zugkraft zu messen. Bevorzugt gelten die angegebenen Dehnungswerte in Faserrichtung. Die Dehnbarkeit verbessert sowohl die Belastbarkeit der Wundauflage als auch deren Vermögen die Beweglichkeit des Patienten im Bereich der Wunde (insbesondere in Gelenkbereichen) aufrechtzuerhalten.

Durch den Vliesaufbau sind im Vliesstoff Öffnungen enthalten, wobei es sich hierbei um Abstände zwischen einzelnen Fasern bzw. Faserfilamenten handelt. Bei den genannten Öffnungen kann es sich um mikroskopische Öffnungen handeln, die mit dem Auge nicht erkennbar sind. Die Öffnungen erlauben den Durchtritt von Flüssigkeit. Sie können dabei in ihrer Größe variieren und müssen nicht einheitlich sein.

Eine bevorzugte Form des Randbereichs, in dem die erste Vliesschicht und die zweite Vliesschicht miteinander verbunden sind, umfasst eine Schweißnaht. Der Begriff Schweißnaht umfasst sowohl eine einzelne Schweißnaht als auch mehrere Schweißnähte. Bevorzugt handelt es sich um eine Schweißnaht, die durch Ultraschallschweißung erzeugt wurde und somit eine ultraschallverschweißte Naht. Die Naht kann eine Breite von bis zu 5 mm, bevorzugt 2 bis 5 mm und besonders bevorzugt 3 bis 4 mm haben. Bevorzugt ist die Naht die einzige Verbindungsart innerhalb des Randbereichs und stellt bevorzugt die einzige dauerhafte Verbindung zwischen der ersten und der zweiten Vliesschicht dar. Sofern die Wundauflage eine optionale dritte Vliesschicht umfasst, kann diese dritte Vliesschicht ebenfalls im Randbereich mithilfe der besagten Schweißnaht mit der ersten sowie der zweiten Vliesschicht verbunden sein.

Ebenfalls bevorzugt ist, dass der besagte Randbereich sowie optional die darin enthaltene Schweißnaht abgerundete Ecken hat. Dies verbessert zum einen das haptische Empfinden für den Patienten, da spitz zulaufende Ecken unangenehm für die Haut sein können und zudem an Kleidungsfasern hängen bleiben können. Zum anderen wird durch abgerundete Ecken die Belastbarkeit der Verbindung zwischen der ersten und der zweiten Vliesschicht verbessert.

Die besagten abgerundeten Ecken sind insbesondere bei einer im wesentlichen
rechteckigen Ausführung (in Draufsicht) des Kissens der Wundauflage empfehlenswert. Es soll jedoch darauf hingewiesen werden, dass die erfindungsgemäße Wundauflage sowie das darin enthaltene Kissen ebenfalls eine runde oder ovale Form (ebenfalls in Draufsicht) haben kann, welche *per se* keine Ecken hat. Runde und ovale Formen verbessern die Anhaftung an konvex geformten Körperbereichen (wie z.B. Ellenbogen oder Ferse). Ist die Wundauflage für die Versorgung im Bereich von Bauch, Rücken, Oberschenkel oder Wade vorgesehen, werden üblicherweise Formen verwendet, die im Wesentlichen rechteckig oder quadratisch sind. Derartige Formen bieten den Vorteil, dass das Volumen der praktisch immer rechteckigen Umverpackungen effizienter genutzt wird.

Weiterhin kann die genannte Wundauflage eine optionale, zusätzliche Deckschicht enthalten. Diese Deckschicht ist an der zweiten (wundabgewandten) Vliesschicht befestigt, so dass sie diese überlagert und eine außenliegende Abschlussschicht bildet. Vorzugsweise liegt die Deckschicht in Form einer Folie vor und kann beispielsweise Polyurethan oder Polyethylen enthalten oder daraus bestehen. Ebenfalls bevorzugt, ist dass die Deckschicht wasserdampfdurchlässig und im Wesentlichen flüssigkeitsundurchlässig ist. Hierdurch entsteht eine Wundauflage, die resistent gegenüber z.B. Spritzwasser ist, aber gleichzeitig ihre Atmungsaktivität beibehält, so dass die bedeckte Haut oder Wunde nicht luftdicht abgeschlossen wird, was ansonsten die Wundheilung beeinträchtigen und eine Mazeration des Gewebes begünstigen könnte. Die Wasserbeständigkeit einer Wundauflage mit Deckschicht übersteigt dabei die Wasserbeständigkeit einer Wundauflage, welche lediglich eine hydrophobe, wasserabweisende zweite Vliesschicht (wundabgewandt) hat. Die Atmungsaktivität der Deckschicht kann erreicht werden, indem die Deckschicht mit Poren versehen wird. Vorzugsweise hat die Deckschicht eine Stärke von 15 bis 25 µm. Es hat sich gezeigt, dass bereits diese Stärke eine optimale Atmungsaktivität mit einem ausreichenden Schutz für die Wundauflage kombiniert und darüber hinaus eine Einsparung von Material ermöglicht.

Befestigt werden kann die Deckschicht an der zweiten Vliesschicht der Wundauflage durch Verwendung eines Adhäsivs. Empfohlen wird die Verwendung eines Acrylklebers. Der Acrylkleber kann dabei z.B. zunächst auf die Unterseite der Deckschicht aufgetragen und die Deckschicht anschließend auf die zweite Vliesschicht aufgeklebt werden. Empfohlen wird das Adhäsiv musterförmig auf die Unterseite der Deckschicht aufzutragen, wobei der unbeschichtete Flächenanteil einen verbesserten Luftaustausch ermöglicht, was die Atmungsaktivität weiter begünstigt. Beispiele für mögliche Beschichtungsmuster sind parallel verlaufende Wellen, eine gitterförmige Anordnung oder Sprenkel in Form von voneinander abgegrenzter distinkter Punkte. Bevorzugt sind 30 bis 50 %, besonders bevorzugt 35 bis 45 % der Fläche der Unterseite der Deckschicht mit Adhäsiv beschichtet.

Die Flächenausdehnung der Decksicht kann derart beschaffen sein, dass andere Bestandteile der Wundauflage überragt werden, so dass ein umlaufender Kleberand ausgebildet wird, der während des Gebrauchs eine Anhaftung der Wundauflage - z.B. an die Haut eines Patienten - ermöglicht. Demgemäß umfasst die vorliegende Erfindung eine Variante der Wundauflage mit umlaufendem Kleberand. Der Kleberand bildet dabei einen Teilbereich der mit einem Adhäsiv beschichteten Deckschicht. In dieser Anordnung stellt die erfindungsgemäße Wundauflage den bereits erwähnten Inselverband dar.

Die im absorbierenden Kissen enthaltene superabsorbierende Substanz kann ein superabsorbierendes Polymer sein und kann in Form von Partikeln und / oder Fasern vorliegen. Diese können bei Kontakt mit Flüssigkeiten aufquellen und dabei ein Gel bilden, welches die Flüssigkeiten bindet. Die Quelleigenschaften der superabsorbierenden Substanz tragen dazu bei, den ersten Vliesstoff mitsamt der antimikrobiellen Zusammensetzung näher an das Wundgewebe heranzuführen, was die Fläche für den Stoffaustausch zwischen der antimikrobiellen Zusammensetzung und dem Wundgewebe maximiert. Die antimikrobielle Wirksamkeit wird dadurch gesteigert.

Vorzugsweise enthält das superabsorbierenden Polymer ein Polyacrylat. Superabsorber auf Basis von Polyacrylat können das besagte Polyacrylat in Form eines Polymers oder als Copolymer enthalten und sind in der Lage große Mengen von Flüssigkeit zu binden, was den absorbierenden Kern mit immenser Absorptionskapazität ausstattet.

Weiterhin kann diese superabsorbierende Substanz innerhalb des Kissens vermischt mit einem oder mehreren anderen hydrophilen, absorbierenden Materialien vorliegen. Bei dem oder den anderen hydrophilen, absorbierenden Materialien kann es sich um Fasern oder Flocken aus Zellulose, Baumwolle und / oder Viskose handeln. Bevorzugt handelt es sich um ein absorbierendes Zellstoffmaterial, besonders bevorzugt um Zellstoffflocken oder Zellstoffwatte und ganz besonders bevorzugt um glattfaserige Zellstoffflocken oder glattfaserige Zellstoffwatte. Typischerweise bestehen die Zellstoffflocken bzw. die Zellstoffwatte aus Zellulosefasern. Am besten handelt es sich um glattfaserige Zellstoffflocken. Der absorbierende Kern kann 30 bis 60 Gew.-% superabsorbierendes Polymer und 40 bis 70 Gew.-% Zellulosefasern enthalten, vorzugsweise enthält der absorbierende Kern 40 bis 50 Gew.-% superabsorbierendes Polymer und 50 bis 60 Gew.-% Zellulosefasern, welche bevorzugt in Form von Zellstoffflocken vorliegen. Zellstoffflocken haben den Vorteil, dass sie während der Produktion einfacher in das Kissen der Wundauflage eingebracht werden können, als lose vorliegende Fasern und sich leichter mit der superabsorbierenden Substanz vermischen lassen. Letzteres führt zu homogenen Absorptionseigenschaften über die gesamte Fläche der Wundauflage und ermöglicht es die gesamte Absorptionskapazität der Wundauflage vollständig auszunutzen. Vorzugsweise ist das superabsorbierende Polymer verteilt und eingebettet in die Zellstoffflocken oder die Zellstoffwatte.

Das Kissen und insbesondere der absorbierende Kern des Kissens kann eine Absorptionskapazität von mindestens 50 g/100 cm², bevorzugt von mindestens 100 g/100 cm² und besonders bevorzugt von mindestens 140 g/100 cm² haben. Diese Absorptionskapazitäten können erreicht werden, durch den Einsatz einer ausreichenden Menge superabsorbierenden Polymers im absorbierenden Kern. Eine empfohlene Menge liegt im bereits erwähnten Bereich von 30 bis 60 Gew.-%. Es ist allerdings auch möglich die Absorptionskapazität des Kissens und insbesondere des absorbierenden Kerns zu begrenzen auf 220 g/100 cm², 250 g/100 cm² oder 300 g/100 cm². Der Vorteil einer Begrenzung der Absorptionskapazität liegt darin, dass manche nässenden Wunden lediglich begrenzte Mengen an Exsudat produzieren und die vollständige Nutzung einer zu hoch gewählten Absorptionskapazität nicht erreicht werden würde, wodurch ein Teil des eingesetzten Materials ungenutzt bliebe.

Es hat sich gezeigt, dass mit einer Ausführung der erfindungsgemäße Wundauflage, die über eine Absorptionskapazität von 140 g/100 cm² bis 220 g/100 cm² verfügt, eine Vielzahl von exsudierenden Wunden optimal versorgt werden können, ohne dass es zu einer nennenswerten Materialverschwendung kommt. Sowohl die Absorptionskapazität des Kissen als auch des absorbierenden Kerns können gemäß des Standards DIN EN 13726-1:2002-06 (Kapitel 3.2) bestimmt werden.

Die erste Vliesschicht der erfindungsgemäßen Wundauflage kann zwei zumindest teilweise miteinander verbundene Einzelvliesschichten beinhalten, so dass die erste Vliesschicht als Doppelvliesschicht vorliegt. Bevorzugt besteht die erste Vliesschicht ausschließlich aus den beiden besagten Einzelvliesschichten. Dabei ist die innere der beiden Einzelvliesschichten die wundabgewandte Einzelvliesschicht und die äußere der beiden Einzelvliesschichten die wundzugewandte Einzelvliesschicht. Die innere und die äußere Einzelvliesschicht bilden die Unterseite des Kissens der Wundauflage aus, wobei die innere Einzelvliesschicht direkten Kontakt zum absorbierenden Kern hat. Die äußere Einzelvliesschicht kann während der Benutzung in direktem Kontakt mit der Wunde stehen.

Bevorzugt besteht die erste Vliesschicht des Kissens der Wundauflage aus zwei vollflächig miteinander verbundenen Einzelvliesschichten. Die beiden Einzelvliesschichten gelten als vollflächig miteinander verbunden, wenn sie vom Anwender optisch und haptisch als eine einzelne Schicht wahrgenommen werden oder wenn eine Einzelvliesschicht nicht - oder nur auf destruktive Weise - von der anderen Einzelvliesschicht angehoben werden kann, so dass beim Anheben ein Leerraum zwischen den beiden Einzelvliesschichten entsteht. Eine solche vollflächige Verbindung erlaubt eine schnelle und ungestörte Flüssigkeitsdurchleitung von der äußeren an die innere Einzelvliesschicht. Darüber hinaus führt die vollflächige Verbindung zu einer höheren Belastbarkeit - insbesondere beim Ablösen einer mit Feuchtigkeit gesättigten Wundauflage von der Wunde.

Die innere und die äußere Einzelvliesschicht können beispielweise thermoplastisch oder mittels Vernadeln miteinander verbunden werden. Letzteres führt zu einem Nadelvliesstoff.

Sofern der erste Vliesstoff der Wundauflagen zwei Einzelvliesschichten umfasst, können sowohl die innere als auch die äußere Einzelvliesschicht unabhängig voneinander jeweils mindestens 30 Gew.-%, bevorzugt mindestens 40 Gew.-%, besonders bevorzugt mindestens 50 Gew.-% und ganz besonders bevorzugt mindestens 60 Gew.-% Kunstfasern enthalten. Die äußere Einzelvliesschicht kann sogar gänzlich aus Kunstfasern bestehen.

Weiterhin kann die innere Einzelvliesschicht ein Flächengewicht von beispielsweise 20 bis 35 g/m² oder 25 bis 30 g/m² haben. Die äußere Einzelvliesschicht kann ein Flächengewicht von beispielsweise 10 bis 30 g/m² oder 12 bis 15 g/m² haben.

Empfohlen wird, dass die beiden Einzelvliesschichten sich in ihrer Beschaffenheit, ihren Eigenschaften, ihrer Struktur und / oder Zusammensetzung zumindest teilweise unterscheiden und somit verschieden sind. Sofern die Verschiedenheit auf unterschiedlicher Zusammensetzung beruht, kann diese beispielsweise durch ganz oder teilweise unterschiedliche Faserarten in den beiden Einzelvliesschichten erreicht werden oder auf unterschiedlichen Anteilen derselben Faserarten beruhen. Dies wird im Folgenden näher ausgeführt:
Empfohlen wird, dass die äußere Einzelvliesschicht Polyamidfasern und / oder
Polypropylenfasern enthält, während die innere Einzelvliesschicht aus einer Mischung aus Polyamidfasern oder Polypropylenfasern zusammen mit Viskosefasern besteht. Durch diese Anordnung entsteht zum einen ein hygroskopischer Gradient, der Flüssigkeiten von der Wunde durch die erste Vliesschicht in den absorbierenden Kern zieht und dort einschließt.

Zum anderen wird die Wahrscheinlichkeit für ein Festkleben der äußeren Einzelvliesschicht an der Wunde durch die hydrophoben Eigenschaften des Materials reduziert.

Besonders empfohlen wird, dass beide Einzelvliesschichten zumindest anteilig dieselbe thermoplastische Kunstfaserart enthalten. Hierdurch kann insbesondere durch thermische Verfahren (z.B. mittels Schweißen) eine besonders stabile Verbindung zwischen der inneren und der äußeren Einzelvliesschicht erzeugt werden. Ein Beispiel hierfür, welches sich besonders bewährt hat, ist die Kombination aus einer äußeren Einzelvliesschicht aus Polypropylenfasern und einer inneren Einzelvliesschicht, die Polypropylenfasern in Kombination mit Viskosefasern enthält. Im Rahmen der vorliegenden Erfindung gilt die Viskosefaser nicht als Kunstfaser oder synthetische Faser.

Eine besonders bevorzugte Variante der erfindungsgemäßen Wundauflage sieht vor, dass sowohl die zweite Vliesschicht als auch die innere und die äußere Einzelvliesschicht der ersten Vliesschicht zumindest anteilig jeweils diese thermoplastische Kunstfaserart enthalten. Es hat sich gezeigt, dass der Einsatz von Polypropylen hierbei besonders gute Ergebnisse erzielt und eine daraus erzeugte Schweißnaht im Randbereich eine überraschend hohe Belastbarkeit aufweist. Auf diese Weise ist es möglich, die Schweißnaht schmal zu halten und / oder die Anzahl der einzelnen Schweißnähte zu reduzieren und somit den effektiv zur Verfügung stehenden Innenraum des Kissens zu vergrößern, so dass der im Kissen befindliche absorbierende Kern entweder mit mehr absorbierendem Material gefüllt werden kann oder aber mehr Raum erhält, um sich in Folge der Flüssigkeitsabsorption auszudehnen. Gleichzeitig führt eine verringerte Schweißnahtfläche zu einer besseren Anschmiegsamkeit der Wundauflage an unebene Hautbereiche und zu einer angenehmeren haptischen Empfindung für den behandelten Patienten.

Weiterhin umfasst die vorliegende Erfindung eine Form der Wundauflage, in welcher die erste Vliesschicht - unabhängig davon, ob diese aus einer oder mehreren Einzelvliesschichten aufgebaut ist - insgesamt 50 bis 70 Gew.-% Polypropylenfasern und 30 bis 50 Gew.-% Viskosefasern enthält. Bevorzugt enthält die erste Vliesschicht 55 bis 65 Gew.-% Polypropylenfasern und 35 bis 45 Gew.-% Viskosefasern. Es hat sich gezeigt, dass eine solche Zusammensetzung ein besonders gutes Ansaugverhalten mit einem besonders guten Durchleitevermögen kombiniert, so dass ein schneller Transport von Flüssigkeiten aus Richtung Wunde in Richtung des absorbierenden Kerns gewährleistet ist. Wenn die zweite Vliesschicht ebenfalls Polypropylenfasern enthält oder aus solchen besteht, können zudem - wie oben erklärt - die erste und die zweite Vliesschicht durch eine besonders belastbare thermoplastische Schweißnaht zwischen den beiden Schichten verbunden werden.

Sofern die erste Vliesschicht Kunstfasern enthält, können diese einer Hydrophilierung unterzogen worden sein, so dass diese Kunstfasern hydrophile Eigenschaften aufweisen. Bei diesen hydrophilierten Kunstfasern kann es sich insbesondere um Polypropylenfasern oder Polyamidfasern handeln. Bei der Hydrophilierung kann es sich um eine chemische oder physikalische Behandlung handeln (z.B. mittels Sauerstoffplasma). Ein Beispiel für eine chemische Hydrophilierung ist die Anwendung von 2-Hydroxyethylmethacrylat und ein Beispiel für eine physikalische Hydrophilierung ist die Anwendung von Sauerstoffplasma. Auf diese Weise kann der Vliesstoff von den vorteilhaften Eigenschaften von z.B. Kunstfasern - wie beispielsweise guter Prozessierbarkeit (z.B. Verschweißbarkeit), geringen Kosten und hoher Beständigkeit - profitieren, und gleichzeitig eine besonders gute Ansaug- und Durchleitefähigkeit aufweisen.

Weiterhin kann die erfindungsgemäße Wundauflage eine erste Vliesschicht enthalten, die als Doppelschicht aus einer inneren und einer äußeren Einzelvliesschicht aufgebaut ist, wobei die äußere, wundseitig ausgerichtete Einzelvliesschicht aus Polypropylenfasern besteht und die erfindungsgemäße antimikrobielle Zusammensetzung trägt und / oder beinhaltet.

Da die erste und die zweite Vliesschicht ähnlich oder zuweilen sogar identisch aussehen können, fungiert die antimikrobielle Zusammensetzung zusätzlich als Indikator bzw. Marker derjenigen Seite der Wundauflage, welche auf die Wunde aufgelegt werden soll. Dabei erscheint die erste Vliesschicht durch die antimikrobielle Zusammensetzung dunkler als die zweite Vliesschicht. Die zweite Vliesschicht kann darüber hinaus farbig ausgestaltet (gefärbt) sein, z. B. grün, um die visuelle Unterscheidbarkeit zu verbessern. Darüber hinaus wird eine Sättigung der Absorptionskapazität der Wundauflage für den Anwender bei einer grünfarbigen zweiten Vliesschicht besser sichtbar, weil eine durchfeuchtete grüne Lage einen stärkeren Kontrast zu einer trockenen grünen Lage aufweist, als dies bei einer weißen Lage der Fall ist.

Ferner kann der absorbierende Kern der erfindungsgemäßen Wundauflage in ein Zellstoffvlies eingeschlagen sein. Dabei kann der absorbierende Kern teilweise oder vollständig in das Zellstoffvlies eingeschlagen sein. Dieses Zellstoffvlies kann derart angeordnet sein, dass es sich zwischen dem absorbierenden Kern und der ersten sowie der zweiten Vliesschicht befindet. Der absorbierende Kern kann durch das Zellstoffvlies derart umwickelt sein, dass das Zellstoffvlies schlauchförmig angeordnet ist. Bevorzugt weist dieser Schlauch aus Zellstoffvlies dabei überlappende Bereiche auf, in welchen Anfang und Ende des Zellstoffvlieses übereinanderliegen und in welchen das übereinanderliegende Zellstoffvlies dauerhaft miteinander verbunden. Besonders bevorzugt sind zudem die Enden des Schlauchs (Ein- und Ausgang des Schlauchs) geschlossen ausgestaltet, wobei dies beispielsweise dadurch erreicht werden kann, dass während des Produktionsprozesses diese überstehenden Schlauchenden zusammengedrückt oder durch Einwirkung von Druck geschnitten werden. Bevorzugt handelt es sich dabei um eine Verkrimpung. Der Vorteil einer Verkrimpung liegt sowohl im Verzicht auf den Einsatz von Chemikalien wie Klebstoffe als auch im Verzicht auf energieintensive Methoden wie z.B. Schweißen. In diesem Sinne kann der absorbierende Kern der erfindungsgemäßen Wundauflage allseitig in ein Zellstoffvlies eingeschlagen sein, wobei dieses Zellstoffvlies bevorzugt teilweise mit sich selbst verkrimpt ist, so dass der absorbierende Kern im Zellstoffvlies fixiert ist.

Das besagte Zellstoffvlies dient innerhalb der Wundauflage als Verteilerschicht, welche die von der ersten Vliesschicht durchgeleitete Flüssigkeit gleichmäßig auf den absorbierenden Kern verteilt (von unten, seitlich und von oben), so dass die superabsorbierende Substanz im absorbierenden Kern gleichmäßig aufquillt, ohne dass das dabei entstehende Gel das weitere Nachströmen von Flüssigkeit ins Kernzentrum blockiert.

Gemäß einer Ausführungsform kann die antimikrobiellen Zusammensetzung die einzelnen Inhaltsstoffe in folgenden Konzentrationsbereichen enthalten: EDTA kann in einer Konzentration von 20 bis 93 Gew.-% oder aber 70 bis 85 Gew.-% vorhanden sein. Auch kann die Konzentration von EDTA mindestens 30 oder mindestens 70 Gew.-% betragen. Silber kann in einer Konzentration von 5 bis 50 Gew.-% oder aber 8 bis 20 Gew.-% vorhanden sein. Auch kann die Konzentration von Silber mindestens 5 oder mindestens 10 Gew.-% betragen. Zink kann in einer Konzentration von 2 bis 50 Gew.-% oder aber 3 bis 20 Gew.-% vorhanden sein. Auch kann die Konzentration von Zink mindestens 3 oder mindestens 10 Gew.-% betragen.

Gemäß einer anderen Ausführungsform ist das Gewichtsverhältnis der Inhaltsstoffe innerhalb der antimikrobiellen Zusammensetzung 80 bis 90 Gew.-% T-EDTA, 5 bis 12 Gew.-% AgNO₃ und 5 bis 15 Gew.-% ZN(NO₃)₂.

Gemäß einer weiteren Ausführungsform enthält die antimikrobielle Zusammensetzung 3 bis 45 Gew.-% eines der folgenden Inhaltsstoffe: ionisches Silber, Silbernitrat, ionisches Zink, Zinknitrat oder Zinksulfat. Gleichzeitig oder auch unabhängig davon kann die antimikrobielle Zusammensetzung 15 bis 95 Gew.-% EDTA enthalten.

Gemäß einer besonders bevorzugten Ausführungsform kann die antimikrobielle Zusammensetzung in Summe 8 bis 12 Gew.-% Silber und Zink enthalten, am besten sind 8 bis 10 Gew.-%.

Das Stoffmengenverhältnis der Stoffmenge von EDTA zur Summe der Stoffmengen aus Ag⁺ und Zn²⁺ kann von 4 zu 1 bis hin zu 4 zu 32 reichen. Ein bevorzugtes Stoffmengenverhältnis von EDTA : Ag⁺ : Zn²⁺ ist 0,9 bis 1,1 : 0,14 bis 0,2 : 0,14 bis 0,2. Die Stoffmengenbestimmung kann in der Standardeinheit mol vorgenommen werden. Das Verhältnis der Inhaltsstoffe zueinander ist ein Faktor, der darüber mitentscheidet, wie groß der Anteil der freien Kationen (Ag+, Zn+) zu den komplexierten und damit an EDTA gebundenen Kationen ist. Dabei hat sich überraschend gezeigt, dass sowohl freie Kationen als auch freie Bindungsstellen am EDTA-Molekül vorteilhaft sind. Vermutet wird, dass eben diese primär für die gewünschten Reaktionen gegen Erreger und Biofilme zur Verfügung stehen. Bevorzugte daraus abgeleitete Bereiche ist ein Gewichtsverhältnis der Gewichtskonzentration von 9 zu 30 bis hin zu 9 zu 36 (9 steht für Teile von EDTA) und ein Stoffmengenverhältnis der Stoffmenge von 4 zu 28 bis hin zu 4 zu 32 (4 steht für Teile von EDTA).

Zur Bereitstellung der antimikrobiellen Zusammensetzung wird empfohlen die Inhaltsstoffe zunächst in einer polaren, ungiftigen Flüssigkeit zu lösen oder zu verteilen. Die Verwendung von Wasser wird hierbei empfohlen, besonders empfohlen wird deionisiertes oder destilliertes Wasser, da es keine störenden Ionen (wie z.B. Na⁺) enthält, die mit den Inhaltsstoffen der antimikrobiellen Zusammensetzung reagieren könnten. Bevorzugt handelt es sich bei der genannten Flüssigkeit um eine flüchtige Substanz, die nach dem Einbringen der antimikrobiellen Zusammensetzung in die Wundauflage ganz oder teilweise verdunstet oder verdampft. Bei späterem Kontakt mit Wundflüssigkeit lösen sich die Inhaltsstoffe der antimikrobiellen Zusammensetzung erneut und werden an die Wunde abgegeben.

Gemäß einer bevorzugten Ausführungsform enthält die polare Flüssigkeit mit der antimikrobiellen Zusammensetzung freie Kationen von Silber und Zink. Dies kann dadurch erreicht werden, dass sowohl Silber als auch Zink jeweils im Überschuss in Relation zu EDTA vorliegen. Beispielsweise kann die Summe der Stoffmengen von Silber und Zink mindestens die dreifache, besser mindestens die fünffache Stoffmenge an EDTA betragen. Die freien Kationen stehen auf diese Weise für biochemische Reaktionen bereit, die sich direkt oder indirekt vorteilhaft auf die Wundheilung auswirken.

So hat sich gezeigt, dass Silber vorrangig eine Wirkung gegenüber pathogenen Einzellern zeigt. Zink ist in der Lage Immunzellen zu aktivieren. Durch seine Funktion als Cofaktor in diversen Transkriptionsfaktoren und enzymatischen Reaktionen fördert Zink den Wundverschluss und schützt Zellen darüber hinaus vor Apoptose, die durch oxidativen Stress oder Bakterientoxine eingeleitet wird. Durch die Bindung von Kalzium kann EDTA vorhandene Biofilme zersetzen und ebenso deren Entstehung unterdrücken.

Die Freisetzung der Kationen (Ag⁺ und Zn²⁺) aus der ersten Vliesschicht der erfindungsgemäßen Wundauflage kann innerhalb von 24 h bei 1 bis 200 µg pro 100 cm² Substrat liegen (in 100 ml Wasser, über 24 h, bei 37°C). Bevorzugt liegt die Freisetzung bei 10 bis 180 µg, noch besser bei 20 bis 160 µg oder bei 30 bis 100 µg.

Überraschenderweise wurde herausgefunden, dass die notwendige Silbermenge innerhalb der antimikrobiellen Zusammensetzung zum Erreichen der antimikrobiellen Wirkung geringer gehalten werden kann, als es zu erwarten gewesen wäre. Es wird vermutet, dass die übrigen Bestandteile EDTA und Zink die Wirkung von Silber potenzieren. Eine reduzierte Silbermenge bietet den Vorteil einer herabgesetzten Cytotoxizität bzw. einer gesteigerten Zellkompatibilität.

Die antimikrobielle Zusammensetzung kann besonders schnell und komfortabel hergestellt werden und zeigt besonders gute Stabilität, wenn die Inhaltsstoffe in Form bestimmter Salze vorliegen. Demgemäß kann die erfindungsgemäße Wundauflage eine antimikrobielle Zusammensetzung umfassen, bei welcher die Inhaltsstoffe in Form von Salzen umfasst sind. Beispielsweise kann Silber in Form von Silbernitrat vorliegen. Zink kann in Form von Zinknitrat oder Zinksulfat vorliegen und EDTA kann in Form von Tetra-Natrium-EDTA vorliegen.

Die "antimikrobielle Zusammensetzung" kann im Rahmen der vorliegenden Erfindung insbesondere in folgenden Zuständen vorliegen:
a) als Mischung aus ionischem Silber und / oder Silbernitrat sowie ionischem Zink und / oder Zinknitrat und / oder Zinksulfat sowie EDTA oder Tetra-Natrium EDTA - sofern nicht anders ausgeführt, liegt die "antimikrobielle Zusammensetzung" primär in dieser Form vor, und zwar bevorzugt in den ionischen Formen
b) als Komplexverbindung Ag₂Zn(EDTA),
c) als Lösung der unter b) genannten Komplexverbindung in einer polaren Flüssigkeit wie Wasser oder einem anderen ungiftigen, polaren Lösungsmittel, oder
d) als Lösung der Salze Silbernitrat, Zinksulfat bzw. Zinksulfatmonohydrat und Tetrasodium EDTA in einer polaren Flüssigkeit wie Wasser oder einem anderen ungiftigen, polaren Lösungsmittel, so dass innerhalb der Lösung die unter b) genannte Komplexverbindung durch Reaktion der genannten Salze untereinander ausgebildet wird. Auch kann das Zinknitrat der antimikrobiellen Zusammensetzung in Form des Hexahydrats Zn(NO₃)₂ x 6H₂O beigemengt werden und das Tetra-Natrium-EDTA als Tetrahydrat oder Dihydrat. Dabei müssen nicht sämtliche Kationen von Silber und Zink zum Komplex abreagieren. Die Ausbildung eines chemischen Gleichgewichts im Sinne einer Dissoziation mit Hin- und Rückreaktion stellt keinen Nachteil dar. Ebenso können auch andere Salze von Silber und Zink als deren Nitrate eingesetzt werden, solange eine Ausbildung der unter b) angegeben Komplexverbindung durch die Reaktanden möglich ist.

Sollten die Inhaltsstoffe der antimikrobiellen Zusammensetzung in der Lösung teilweise ausfallen, stellt dies nicht automatisch einen Nachteil für deren spätere Wirksamkeit dar. In einem solchen Fall wird empfohlen, die ungelösten Partikel unmittelbar vor dem Einbringen in die Wundauflage durch Rühren oder Schütteln in eine möglichst homogene Suspension zu überführen.

Gemäß einer bevorzugten Ausführungsform trägt die Wundauflage 17 bis 32 g der antimikrobiellen Zusammensetzung pro m² wundzugewandter Oberfläche oder Wundkontaktfläche. Noch bessere Ergebnisse sind zu erwarten im Bereich von 20 bis 26 g / m².

Auch kann die antimikrobielle Zusammensetzung in einer Menge enthalten sein, dass 2 bis 5 Gew.-% der Wundauflage, der ersten Vliesschicht oder der Wundkontaktschicht durch die antimikrobielle Zusammensetzung gestellt werden. Beispielsweise kann eine Wundauflage oder Vliesschicht oder Wundkontaktschicht, die 50 g wiegt, 1 bis 2,5 g antimikrobielle Zusammensetzung enthalten. Noch bessere Ergebnisse sind im Bereich von 3 bis 4 Gew.-% zu erwarten.

Gemäß einer besonders bevorzugten Ausführungsform enthält die Wundauflage die antimikrobiellen Inhaltsstoffe innerhalb der antimikrobiellen Zusammensetzung in einem Gewichtsverhältnis von 80 bis 92 Gew.-% EDTA zu 5 bis 10 Gew.-% Silber zu 3 bis 10 Gew.-% Zink vorliegen. Diese Ausführungsform bietet eine besonders gute Biokompatibilität bedingt durch eine äußerst geringe Cytotoxizität. Die Biokompatibilität ist ganz besonders gut, wenn die antimikrobielle Zusammensetzung mit dem besagten Gewichtsverhältnis der Inhaltsstoffe 2 bis 3 % des Gewichts der ersten Vliesschicht, der Wundkontaktschicht oder des Gesamtgewichts der Wundauflage ausmacht.

Vorteilhafterweise enthält die erfindungsgemäße Wundauflage die antimikrobielle Zusammensetzung innerhalb der ersten Vliesschicht. Insbesondere kann die erste Vliesschicht mit der Zusammensetzung beschichtet, imprägniert oder getränkt sein. Falls die erste Vliesschicht als Doppelvliesschicht vorliegt, sollte zumindest die äußere der beiden Einzelvliesschichten mit der antimikrobiellen Zusammensetzung ausgestattet sein.

Das Imprägnieren und das Tränken führen zu einem zumindest teilweisen, besser zu einem vollständigen Durchdringen der Faserstruktur der ersten Vliesschicht mit der antimikrobiellen Zusammensetzung. Dies bietet den Vorteil einer Depotwirkung und verringert gleichzeitig die Wahrscheinlichkeit, dass sich Krankheitserreger in der Wundauflage vermehren können. Das Tränken der Wundauflage bzw. des Vlieses der Wundauflage kann beispielsweise mittels Tauchverfahren erfolgen, während das Imprägnieren beispielsweise durch Aufsprühen erfolgen kann. Andere Techniken sind ebenfalls möglich. So kann die Wundauflage mittels einer Walze oder durch Bestreichen (z.B. mittels Pinsel) mit der antimikrobiellen Zusammensetzung beschichtet werden. Nach erfolgter Aufbringung kann das Lösungsmittel entfernt werden. Dies kann beispielsweise mittels aktiver oder passiver Trocknung oder Verdampfen geschehen.

Überraschenderweise hat sich gezeigt, dass der mit der antimikrobiellen Zusammensetzung ausgestattete erste Vliesstoff der erfindungsgemäßen Wundauflage durchleitend für Wundexsudat und andere Flüssigkeiten ist.

Die antimikrobielle Zusammensetzung und somit die damit ausgestattete Wundauflage haben antimikrobielle, insbesondere antibakterielle Eigenschaften, wobei die antibakteriellen Eigenschaften eine Wirkung gegen die humanpathogenen Keime S. *aureus* sowie P. *aeruginosa* umfassen. Die antimikrobielle Wirkung tritt bereits beim Initialkontakt mit den Erregern ein und kann sich im Laufe der Zeit verstärken, wobei eine Kontaktzeit (z.B. Anwendungsdauer auf einer Wunde) von 1 bis 72 Stunden oder 1 bis 24 Stunden bevorzugte Anwendungsdauern sind.

Das Einbringen der antimikrobiellen Zusammensetzung in die erste Vliesschicht bewirkt eine räumliche Trennung der Metalle von der superabsorbierenden Substanz des absorbierenden Kerns. Dies stellt sicher, dass Metalle, welche in Form von positiv geladenen, gelösten Kationen vorliegen, nicht durch elektromagnetische Wechselwirkung an die negativ geladene Acrylsäure, welche Teil der superabsorbierenden Substanz sein kann, gebunden werden. Denn durch eine solche unerwünschte Interaktion würden gebundene Kationen nicht mehr für die vorgesehenen Reaktionen der Erregerabwehr oder der Heilungsförderung zur Verfügung stehen. In diesem Sinne kann im Rahmen der vorliegenden Erfindung vorgesehen sein, dass der absorbierende Kern der Wundauflage kein Zn und Ag enthält.

Gemäß einer bevorzugten Ausführungsform enthält die Wundauflage die bereits erwähnte dritte Vliesschicht. Die dritte Vliesschicht überlagert die erste Vliesschicht wundseitig. Dadurch wird die antimikrobielle Zusammensetzung bedeckt. Auf diese Weise wird die antimikrobielle Zusammensetzung zusätzlich geschützt und eine mögliche ungewollte Anhaftung an einem adhäsiven Release-Liner wird verhindert. Auch einem möglichen Abfärben der antimikrobiellen Beschichtung an z.B. Kleidung oder Hände des medizinischen Personals wird auf diese Weise entgegengewirkt. Bevorzugt hat die dritte Vliesschicht dieselbe chemische Zusammensetzungen, denselben Aufbau und dieselbe Struktur wie die erste Vliesschicht. Demgemäß sind alle oben in Zusammenhang mit der ersten Vliesschicht erwähnten Merkmale auch für die dritte Vliesschicht anwendbar. Insbesondere kann vorgesehen sein, dass die erste Vliesschicht und die dritte Vliesschicht identisch sind.

Darüber hinaus kann das Kissen eine zusätzliche perforierte Silikonschicht aufweisen, die an der ersten Vliesschicht oder aber - falls vorhanden - dritten Vliesstoffschicht befestigt ist. Eine solche Silikonschicht kann die atraumatischen Eigenschaften des Kissens weiter verbessern. Das Silikon der Silikonschicht kann als Silikongel vorliegen, welches adhäsive Eigenschaften hat. Insbesondere kann diese Silikonschicht eine perforierten Folie umfassen, wobei eine der ersten oder dritten Vliesschicht des Kissens zugewandte Seite der Folie mit einem Acrylkleber und eine gegenüberliegende Seite derselben Folie mit einem Silikongel beschichtet ist. In Verwendung berührt das Kissen die Wunde also mit dem atraumatischen Silikongel. Vorzugsweise besteht die Folie aus Polyurethan. Die erwähnten Perforationen sind in diesem Fall Aussparungen in Folie, Silikongel und Acrylkleber. Die Perforationen ermöglichen es dem Wundexsudat, durch die Silikonschicht in das absorbierende Kissen zu gelangen. Gleichzeitig können die Inhaltsstoffe der antibakteriellen Zusammensetzung durch die Perforationen in die Wunde gelangen. Die Perforationen können beispielsweise kreisförmig sein und einen Durchmesser von 2,3 bis 2,5 mm aufweisen. Die durch die Perforationen erzeugte offene Fläche kann beispielsweise 18 bis 27 % betragen. Die Folie kann eine Stärke von 10 bis 20 µm haben.

Weiterhin kann im Rahmen der vorliegenden Erfindung die erste bzw. dritte Vliesschicht oder die in der ersten Vliesschicht optional vorhandenen Einzelvliesschichten gegebenenfalls frei sein von bestimmten Substanzen. Beispielsweise kann der Vliesstoff frei sein von Gelatine und / oder Kollagen oder generell frei sein von Substanzen tierischen Ursprungs (z.B. Chitosan). Vliese, die frei sind von derartigen Stoffen zeigen im Allgemeinen eine längere Lagerungsbeständigkeit und ein geringeres Allergiepotential. Zudem kann eine unerwünschte Interaktion derartiger zusätzlicher Substanzen mit der antimikrobiellen Zusammensetzung ausgeschlossen werden.

Darüber hinaus kann vorgesehen sein, dass die erfindungsgemäße Wundauflage keine Hydrogele, Schäume oder gewebten, gewirkten oder gestrickten Textilien enthält.

Vor der Verwendung kann die untere (wundzugewandte) Seite der Wundauflage durch eine Schutzschicht wie beispielsweise einen Release-Liner abgedeckt sein. Eine solche Schutzschicht kann in Form einer Folie ausgestaltet sein und wird vom Anwender unmittelbar vor der Verwendung entfernt.

Die erfindungsgemäße Wundauflage ist sterilisationsbeständig und behält ihre funktionellen und strukturellen Eigenschaften nach der Sterilisation bei. Insbesondere bleiben die antimikrobiellen Eigenschaften erhalten. Die Sterilisation kann beispielsweise mittels Ethylenoxid, Dampfsterilisation (Autoklavierung) oder Heißluftsterilisation erfolgen.

Darüber hinaus umfasst die Erfindung auch ein Kit umfassend
a) eine erfindungsgemäße Wundauflage wie sie hierin beschrieben ist inklusive der antimikrobiellen Zusammensetzung
b) ein Befestigungsmittel, wobei das Befestigungsmittel geeignet ist zur Befestigung der Wundauflage an einer Wunde. Bei dem Befestigungsmittel kann es sich um eine Klebefolie handeln. Bei dem Kit kann es sich um eine Verpackung oder ein Set handeln.

Ein weiterer Aspekt der Erfindung betrifft die erfindungsgemäße Wundauflage zur Anwendung in einem Verfahren zur Behandlung von Wunden, bevorzugt von infizierten Wunden, besonders bevorzugt von Wunden, die mit *S. aureus* und / oder *P. aeruginosa* infiziert sind oder in einem Verfahren zur Wundtherapie und / oder zur Reduktion der Keimzahl in Wunden und / oder zur Prävention von Wundinfektionen. Hierbei kann vorgesehen sein, dass die Anwendung über einen Zeitraum von 0,1 bis 24 Stunden stattfindet oder dass die Anwendung in einem Zeitraum von mindestens 24 Stunden stattfindet, wobei letzteres beispielswiese bei bereits stark ausgeprägten Infektionen oder bei Patienten mit Immunschwäche geboten sein kann. Bei den Wunden kann es sich insbesondere um Ulzera, traumatische Wunden (inklusive Schnitt- und Operationswunden), chronische Wunden, blutende Wunden, eiternde Wunden, nekrotische Wunden, belegte (fibrinhaltige) Wunden und exsudierende (nässende) Wunden handeln.

### Figuren

Im Folgenden werden die Figuren näher erläutert.

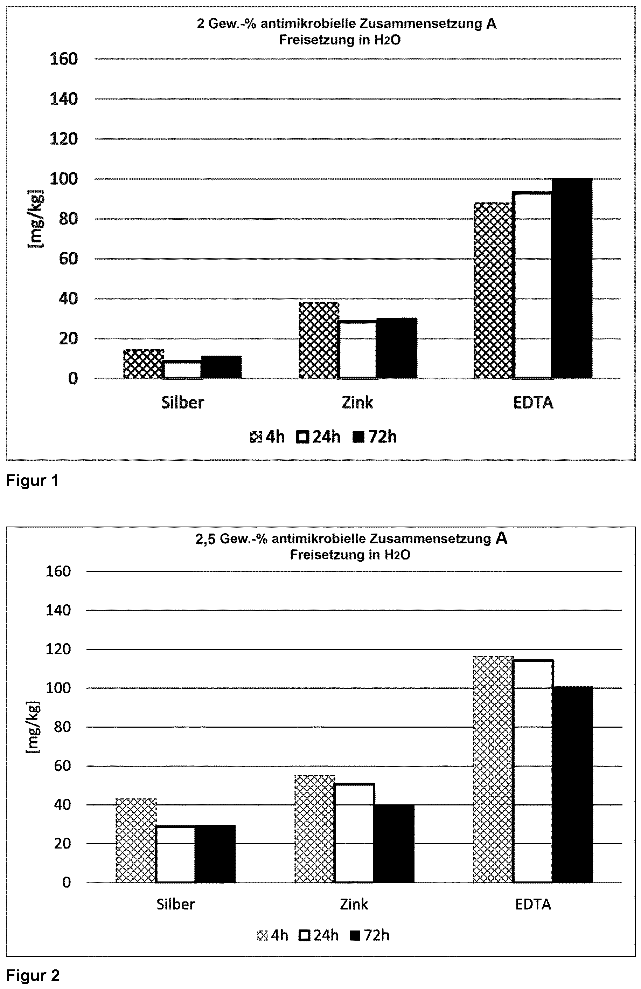

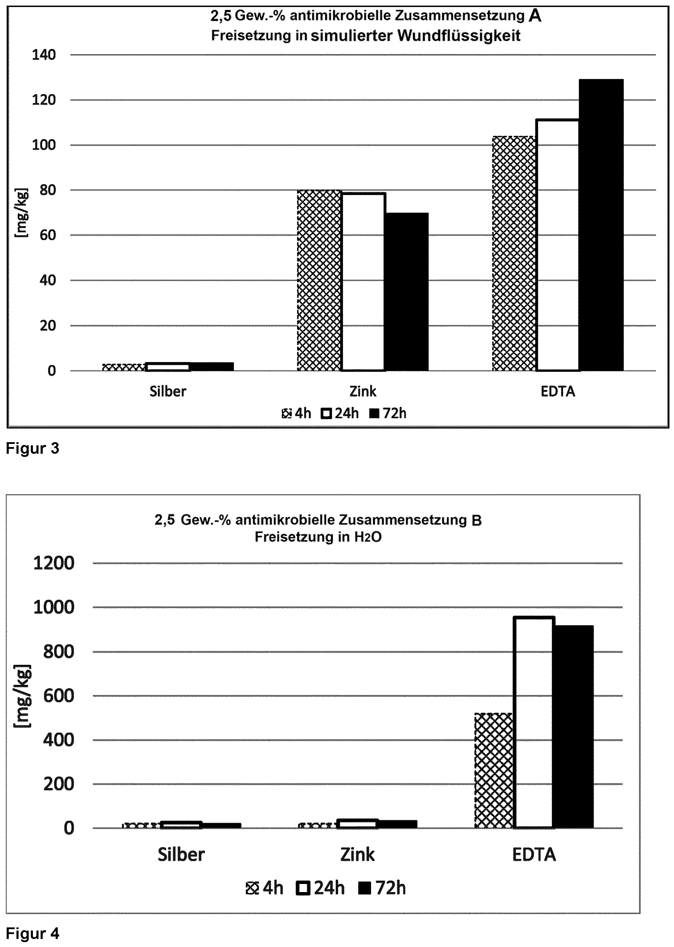

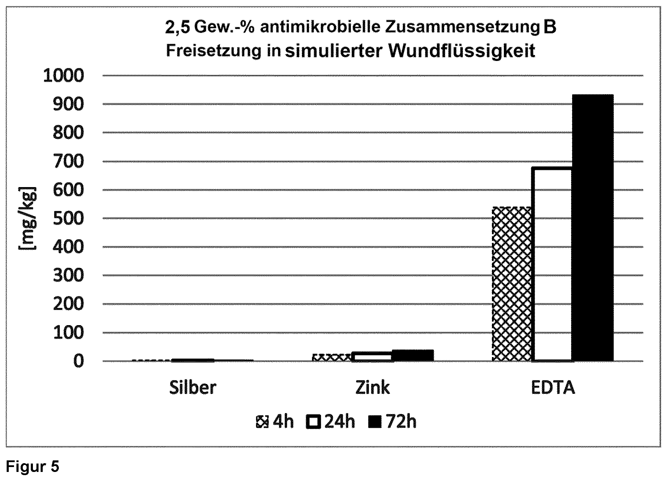

Die Fig. 1 - 5 zeigen die Freisetzung der antimikrobiellen Zusammensetzung aus Wundauflagen. Bei diesen Freisetzungsversuchen wurden Messwerte nach 4h, 24h und 72h aufgenommen, welche die Konzentrationen der einzelnen antibakteriellen Inhaltsstoffe Ag, Zn und EDTA in mg pro kg des jeweiligen Lösungsmittels zeigen:
Fig. 1 zeigt die Freisetzung der antimikrobiellen Zusammensetzung A (s. Beispiel 1) aus einer Wundauflage an Wasser. Die Wundauflage enthielt diese Zusammensetzung in einer Menge von 2 % ihres Eigengewichts.
Fig. 2 zeigt die Freisetzung der antimikrobiellen Zusammensetzung A (s. Beispiel 1) aus einer Wundauflage an Wasser. Die Wundauflage enthielt diese Zusammensetzung in einer Menge von 2,5 % ihres Eigengewichts.
Fig. 3 zeigt die Freisetzung der antimikrobiellen Zusammensetzung A (s. Beispiel 1) aus einer Wundauflage an simulierte Wundflüssigkeit. Die Wundauflage enthielt diese Zusammensetzung in einer Menge von 2,5 % ihres Eigengewichts.
Fig. 4 zeigt die Freisetzung der antimikrobiellen Zusammensetzung B (s. Beispiel 1) aus einer Wundauflage an Wasser. Die Wundauflage enthielt diese Zusammensetzung in einer Menge von 2,5 % ihres Eigengewichts.
Fig. 5 zeigt die Freisetzung der antimikrobiellen Zusammensetzung B (s. Beispiel 1) aus einer Wundauflage an simulierte Wundflüssigkeit. Die Wundauflage enthielt diese Zusammensetzung in einer Menge von 2,5 % ihres Eigengewichts.
Die Fig. 6 - 7 zeigen die antibiotische Wirkung von Wundauflagen mit der antimikrobiellen Zusammensetzung B.

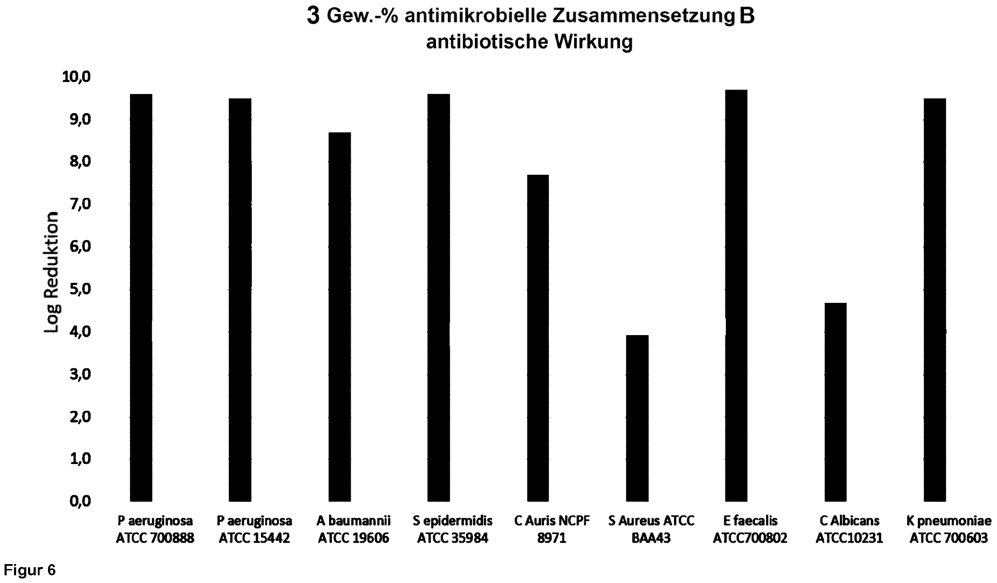

Fig. 6 zeigt die antibiotische Wirkung von Wundauflagen mit der antimikrobiellen Zusammensetzung B (s. Beispiel 1) gegenüber ausgewählten pathogenen Mikroorganismen nach einer Kontaktdauer von 24 h. Jede Wundauflage enthielt die antimikrobiellen Zusammensetzungen in einer Menge von 3% ihres Eigengewichts. Die Skala der Ordinate zeigt die Abnahme der Organismenzellzahl mithilfe des logarithmischen Maßstabs.

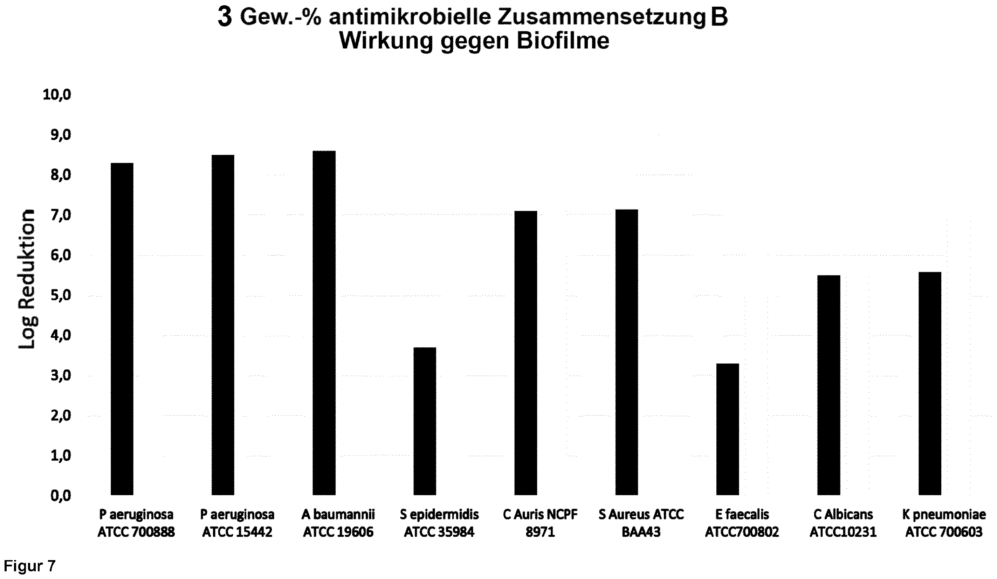

Fig. 7 zeigt die Wirkung von Wundauflagen mit der antimikrobiellen Zusammensetzung B (s. Beispiel 1) gegenüber Biofilmen ausgewählter pathogener Mikroorganismen nach einer Kontaktdauer von 24 h. Jede Wundauflage enthielt die antimikrobiellen Zusammensetzungen in einer Menge von 3% ihres Eigengewichts. Die Skala der Ordinate zeigt die Abnahme der Organismenzellzahl mithilfe des logarithmischen Maßstabs.

### Beispiel 1: Bereitstellung von antimikrobiellen Zusammensetzungen

Das verwendete Wasser war demineralisiert.

### 1.1 Antimikrobielle Zusammensetzung mit Kationenüberschuss (Zusammensetzung A)

Bei dieser Variante der antimikrobiellen Zusammensetzung lag das molare Verhältnis von EDTA zu Zink zu Silber bei 11 : 44,5 : 44,5. Diese Zusammensetzung wurde gemäß dem folgenden Ablauf angefertigt:
Zunächst wurden drei einzelne Lösungen von Tetra-Natrium-EDTA (T-EDTA), AgNO₃ und
ZnNO₃ hergestellt. Hierzu wurden
   - 0,8 g T-EDTA in 6,2 g Wasser
   - 1,45g AgNO₃ in 5,55 g Wasser und
   - 2,53 Zn(NO₃)₂ x 6 H₂O in 25,47 g Wasser
   eingerührt, bis sich jeweils eine klare und farblose Lösung gebildet hat.

Anschließend wurde die Lösung von T-EDTA mit der Lösung von ZnNO₃ durchmischt. Zu guter Letzt wurde die Lösung von AgNO₃ zugegeben und durchmischt, so dass alle drei Inhaltsstoffe der antimikrobiellen Zusammensetzung in einer Lösung vorlagen.

Die derart hergestellte wässrige Lösung hatte eine Gesamtsalzkonzentration von etwa 9%.

### 1.2 Zusammensetzung mit Überschuss an EDTA (Zusammensetzung B)

Bei dieser Variante der antimikrobiellen Zusammensetzung lag das molare Verhältnis von EDTA zu Zink zu Silber bei: 78 : 11 : 11. Durch den im Vergleich zu Beispiel 1.1 verminderten Silberanteil zeigt diese Zusammensetzung eine bessere Zellverträglichkeit gegenüber menschlichem Gewebe.

Diese Zusammensetzung wurde gemäß dem folgenden Ablauf angefertigt:
Zunächst wurden drei einzelne Lösungen von Tetra-Natrium-EDTA (T-EDTA), AgNO₃ und
ZnNO₃ hergestellt. Hierzu wurden
   - 2,8 g T-EDTA in 5,5 g Wasser
   - 0,18 g AgNO₃ in 3,5 g Wasser und
   - 0,32g Zn(NO₃)₂ x 6 H₂O in 12 g Wasser
   eingerührt, bis sich jeweils eine klare und farblose Lösung gebildet hat.

Anschließend wurde die Lösung von T-EDTA mit der Lösung von ZnNO₃ durchmischt. Zu guter Letzt wurde die Lösung von AgNO₃ zugegeben und durchmischt, so dass alle drei Inhaltsstoffe der antimikrobiellen Zusammensetzung in einer Lösung vorlagen.

Die derart hergestellte wässrige Lösung hatte eine Gesamtsalzkonzentration von etwa 14%.

### 1.3 Weitere Zusammensetzungen

Analog zu den bei Beispielen 1.1 und 1.2 wurden antimikrobielle Zusammensetzungen mit dem folgenden Verhältnis an Inhaltsstoffen hergestellt:
Zusammensetzung C = 33 EDTA : 33 Ag : 34 Zn
Zusammensetzung D = 55,5 EDTA : 34,5 : Ag : 10 Zn

### Beispiel 2: Ausstattung von Wundauflagen mit antimikrobiellen Zusammensetzungen

### 2.1 Ausstattung von Vliesschichten mit den antimikrobiellen Zusammensetzungen

Bereitgestellt wurden Vliesschichten mit einer Fläche von 11 cm x 11 cm bestehend aus jeweils zwei Einzelvliesschichten: Eine nach unten (in Benutzung wundseitig) ausgerichtete Einzelvliesschicht aus Polypropylenfasern und eine nach oben (in Benutzung von der Wunde abgewandt) ausgerichtete Einzelvliesschicht aus 64 Gew.-% Polypropylenfasern und 36 Gew.-% Viskosefasern. Die beiden Einzelvliesschichten waren durch den Einsatz von Hitze und unter Nutzung der thermoplastischen Eigenschaften der Polypropylenfasern vollflächig miteinander verbunden. Jede resultierende (Doppel-)Vliesschicht hatte eine Masse von etwa 0,54 g.

Die bereitgestellten Vliesschichten wurden entweder in die Lösung der Zusammensetzung A (gemäß Beispiel 1.1) oder der Zusammensetzung B (gemäß Beispiel 1.2) für 30 Sekunden eingelegt (Tauchverfahren) und anschließend zwischen zwei Lagen Papiertüchern zusammengerollt, um überschüssige Flüssigkeit herauszupressen.

Die mit Zusammensetzung A bzw. B behandelten Vliesschichten wurden durch das Herauspressen der Flüssigkeit auf ein solches Gewicht eingestellt, dass die Salze innerhalb der später generierten (trockenen) Wundauflagen jeweils 2-Gew.-%, 2,5 Gew.-% und 3 Gew.-% der fertigen Wundauflagen ausmachten.

Im Falle der anvisierten Endkonzentration von 3 Gew.-% Salzen in der Wundauflage entsprach dies einem Gewicht von 2,92 g der ausgepressten Vliesschicht, wovon 2,38 g Salzlösung waren. Aufgrund der 14%igen Lösungskonzentration lag die reine Salzmasse innerhalb der Vliesschicht bei etwa 0,33 g. Die restliche (zu diesem Zeitpunkt noch fehlende) Masse wurde später durch Hinzufügen der übrigen Strukturen der Wundauflage (siehe 2.2) hinzugefügt, wodurch die anvisierten 3 Gew.-% Salze innerhalb der fertigen Wundauflage erreicht wurden.

Die behandelten und durch Auspressen auf das entsprechende Zielgewicht eingestellten Vliesschichten wurden in einem Trocknungsofen bei Umluft und 60°C für etwa 1h getrocknet. Dabei dienten geöffnete Petrischalen, auf welche die Vliesschichten platziert wurden als Abstandshalter, um einen direkten Kontakt mit dem Trocknungsofen zu vermeiden.

### 2.2 Einbau von antimikrobiell behandelten Vliesschichten in Wundauflagen

Die gemäß Beispiel 2.1 mit antimikrobiellen Zusammensetzungen ausgestatteten Vliesschichten wurden durch Schneiden verkleinert, so dass deren Flächen 3 cm x 3 cm betrugen und anschließend mit anderen Bestandteilen zu Kissen zusammengebaut, wobei die antimikrobiell behandelten Vliesschichten als erste Vliesschichten (in Benutzung wundseitig ausgerichtet) in die Kissen eingebaut wurden. Als zweite Vliesschicht (in Benutzung wundabgewandt ausgerichtet) diente ein hydrophobes Vliesmaterial aus Polypropylenfasern, welches ebenfalls eine Fläche von 3 cm x 3 cm hatte. Als absorbierender Kern innerhalb des Kissens dienten Zellstoffflocken, in welche superabsorbierende Polymerpartikel eingebettet waren.

Die anderen Bestandteile des Kissens hatten eine Masse von etwa 0,72 g. Zur Einstellung auf eine Konzentration der antimikrobiellen Zusammensetzung in der Wundauflage von beispielsweise 3-Gew.-% wurden diese Bestandteile mit einer behandelten, rückgetrockneten Vliesschicht mit einem Gewicht von etwa 0,064 g (davon etwa 0,024 g Salze) kombiniert. Die erste und die zweite Vliesschicht wurden in ihrem umlaufenden Randbereich, in welchem sie sich direkt berührten, durch Einwirkung von Hitze miteinander verbunden und das Kissen auf diese Weise verschlossen.

Die Größe von 3 cm x 3 cm erlaubte die spätere Prüfung der antimikrobiellen Wirksamkeit in Petrischalen. Für den praktischen Einsatz in der Wundbehandlung können Wundauflagen mit einer größeren Fläche, nach demselben Prinzip wie in diesem Beispiel erläutert, hergestellt werden.

### Beispiel 3: Bestimmung der Cytotoxizität

Die Bestimmung der Cytotoxizität fand gemäß des Standards ISO10993-5 - Abschnitt 8.3 "Prüfung durch direkten Kontakt" - statt. Die Zellviabilität wurde mittels des Invitrogen^{™} CyQUANT^{™} Zellproliferations-Assaykits von Thermo Fisher Scientific bestimmt. Die für den Test verwendeten Zellen stammten aus der Zelllinie L929 (Mausfibroblasten; ECACC Nr. 88102702). Die Zellviabilität wurde als prozentueller Vergleichswert in Relation zu Kontrollzellen in Dulbecco's Modified Eagle Medium (DMEM) ermittelt. Als Prüflinge fungierten Vliesschichten, welche aus zwei Einzelvliesschichten zusammengesetzt waren (Bereitstellung gemäß Ausführungsbeispiel 2.1). Die Vliesschichten hatten jeweils eine Größe von 1 x 1 cm. Die Ergebnisse sind in der nachfolgenden Tabelle dargestellt:

**Tab. 1: Ergebnisse der Cytotoxizitätsversuche**

| Gew.-% antimikrobieller Zusammensetzung in Wundauflage | Art der Zusammensetzung [A, B, C, D] | Verhältnis EDTA : AG : ZN | Qualitätsstufe | Zellviabilität [% im Vergleich zur Kontrolle in DMEM ] |
|---|---|---|---|---|
| 2 | A | 11 : 44,5 : 44,5 | 3 | 65 |
| 2 | C | 33: 33: 34 | 3 | 57 |
| 2 | B | 78 : 11 : 11 | 3 | 69 |
| 2 | D | 55,5: 34,5 : 10 | 3 | 55 |
| 2,5 | B | 78 : 11 : 11 | 3 | 71 |
| 2,5 | A | 11 : 44,5 : 44,5 | 3 | 51 |
| 3 | B | 78 : 11 : 11 | 3 | 34 |
| 0 | n.a. | n.a. | 0 | 97 |

### Beispiel 4: Freisetzungsversuche

Getestet wurde die Freisetzung der antimikrobiellen Inhaltsstoffe aus Wundauflagen mit unterschiedlicher antibakterieller Zusammensetzung. Als Testmedien (11 ml / Prüfling) fungierten:
I) dest. Wasser
II) simulierte Wundflüssigkeit (50% bovines Serum ; 50% wässrige Peptonlösung)

**Tab. 2 Prüflinge:**

| Gew.-% antimikrobieller Zusammensetzung in Wundauflage | Art der Zusammensetzung [A, B, C, D] | Verhältnis EDTA : AG : ZN | Anzahl getesteter Prüflinge | Testmedium |
|---|---|---|---|---|
| 2 | A | 11 : 44,5 : 44,5 | 6 | I |
| 2,5 | A | 11 : 44,5 : 44,5 | 6 | I + II |
| 2,5 | B | 78 : 11 : 11 | 6 | I + II |

Die Prüflinge wurde in dem jeweiligen Testmedium bei 37°C unter konstanter Bewegung (120 rpm) inkubiert. Die Inkubationszeiträume betrugen 4h, 24h und 72h.

Nach Ablauf des jeweiligen Zeitraums wurden 4 ml der Flüssigkeit entnommen und die darin enthaltene Konzentration an EDTA bestimmt. Die restliche Flüssigkeit wurde mit 20 µl Salpetersäure (65%) versetzt und zur Bestimmung der Konzentration an Ag⁺ und Zn²⁺ verwendet. Die Bestimmung der EDTA-Konzentration erfolgte mittels High Performance Liquid Chromatography (HPLC-UV / DAD). Die Bestimmung der Kationenkonzentration erfolgte mittels optischer Emissionsspektrometrie mit induktiv gekoppeltem Plasma gemäß DIN EN ISO 11885.

Die Ergebnisse sind in Fig. 1 - 5 dargestellt.

Anhand der Graphen ist erkennbar, dass in einigen Fällen die Messwerte der in Lösung übergegangenen Inhaltsstoffe der antimikrobiellen Zusammensetzung nach einer initialen Freisetzung (Wert nach 4h) wieder abnehmen (Werte nach 24h und 72h). Mögliche Ursachen hierfür sind eine Abscheidung an der Wand des Glasgefäßes, eine Rückabsorption in die Wundauflage sowie eventuelle Messschwankungen.

Insgesamt kann festgestellt werden, dass alle getesteten Mengen (2 und 2,5 Gew.-%) der zwei antimikrobiellen Zusammensetzungen (A und B) in beiden Flüssigkeiten (Wasser und simulierte Wundflüssigkeit) zu einer deutlichen Freisetzung der antimikrobiellen Inhaltsstoffe (Ag, Zn, EDTA) geführt haben.

### Beispiel 5: Nachweis der antimikrobiellen Wirksamkeit von Wundauflagen nach AATCC 100

Als Testmuster zur Bestimmung der antimikrobiellen Wirksamkeit dienten Wundauflagen, die gemäß Beispiel 2 hergestellt wurden. Diese enthielten die antimikrobielle Zusammensetzung B, und zwar in einer Menge von 3-Gew.-% der Wundauflage. Die antimikrobielle Zusammensetzung befand sich in der ersten Vliesschicht.

Der Versuch erfolgte im Kontakttest in Anlehnung an den Standard AATCC 100 nach folgendem Schema:
Eine Übernachtkultur des jeweiligen Mikroorganismus wurde angesetzt durch Inokulation von 10 ml TSB mit einer Einzelkolonie. Die nachfolgende Inkubation fand bei 37°C und 125 rpm auf einem Schüttelinkubator statt. Die Übernachtkulturen wurden auf 1 x 10⁸ CFU / ml eingestellt.

Die Wundauflagen wurden pro Erreger in dreifacher Ausführung getestet. Dazu wurden die Wundauflagen in Petrischalen überführt (eine Wundauflagen pro Petrischale) und mit 0,85 %-iger NaCl-Lösung benetzt. Anschließend wurden die Wundauflagen einzeln in 50 ml Falcon Röhrchen eingebracht, mit 10 ml TSB versetzt und mit 100 µl der Übernachtkultur angeimpft, so dass die Sollkonzentration bei 1 x 10⁶ CFU/ml lag.

Die Wundauflagen wurden bei 37°C und 125 rpm auf einem Schüttelinkubator bebrütet. Anschließend wurde 1 ml jeder Probe in neue 50 ml Falcon Röhrchen überführt und 9 ml Neutralisationslösung zugeführt.

Der nachfolgende Schritt erfolgte in einer 96-Well Platte, wobei 200 µl Zelllösung in die Wells der ersten Plattenspalte eingefüllt wurden. In die Wells der Plattenspalten 2 bis 7 wurden 180 µl hinzugeführt. Nun wurde eine 10-fache serielle Verdünnung der Zelllösung angesetzt, indem 20 µl der Lösung in die jeweils nachfolgenden Wellspalten übertragen wurden.

Von jeder Verdünnung wurden 50 µl in zweifacher Ausführung auf TSA-Agarplatten ausplattiert und bei 37 °C über Nacht inkubiert. Am folgenden Tag wurden die Kolonien ausgezählt.

Die Ergebnisse sind in Fig. 6 als Mittelwerte dargestellt.

### Beispiel 6: Nachweis der Wirksamkeit erfindungsgemäßer Wundkontaktschichten gegenüber Biofilmen mittels Drip-Flow-Bioreaktor

Es wurde die Wirkung erfindungsgemäßer Wundauflagen an Biofilmen getestet, die im Drip-Flow-Reaktor erzeugt wurden. Die Tests fanden in Anlehnung an den Standard ASTM y - 13 statt. Allerdings mit minimalen Abwandlungen, um Daten ausgehend von verschiedenen Mikroorganismenspezies generieren zu können. Diese eingesetzten Organismen lassen sich der folgenden Tabelle entnehmen:

**Tab. 3 Biofilmerzeugende Organismen**

| **grampositive Organismen** | | **Hinterlegungs-Nr. bzw. Stamm** |
|---|---|---|
| | Staphylococcus aureus | ATCC 29213, MRSA ATCC BAA-43 |
| | Staphylococcus epidermidis | ATCC 35984 |
| | Enterococcus faecalis | ATCC 29212 |

| **gramnegative Organismen** | | |
|---|---|---|
| | Pseudomonas aeruginosa | ATCC 15442 |
| | | ATCC 700888 |
| | Acinetobacter baumannii | ATCC 19606 |
| | Klebsiella Pneumoniae | ATCC 700603 |
| | | |

| **pilzliche Organismen** | | |
|---|---|---|
| | Candida albicans | ATCC 10231 |
| | Candida auris | NCPF 8971 |

Die Test erfolgten gemäß folgendem Protokoll: Zunächst wurden 10 ml TSB mit einer einzelnen Kolonie eines jeden Stammes angeimpft und bei 37 °C und 125 rpm auf einem Schüttelinkubator als Übernachtkultur angesetzt. Absorbierende Pads wurden mittels Klebstoff an den Objektträgern befestigt und die Kammern des Drip Flow Reaktors mit den Objektträgern bestückt. Anschließend wurde der Reaktor im Autoklaven sterilisiert.

Die absorbierenden Pads wurden mit 1 ml TSB befeuchtet und mit Polycarbonatfiltern bestückt (2 cm x 2 cm ; 0,2 µm). Die Übernachtkulturen wurden auf 1 x 10⁸ CFU/ml eingestellt. Anschließend wurden die Filter mit 10 µl dieser Lösung benetzt und trocknen lassen.

Die Abdeckungen des Reaktors wurden befestigt und der Reaktor mit einem 10 I Glasballon (270 mg/I TSB) befestigt. Der Schlauch für die Nährstofflösung wurde an eine Pumpe angeschlossen und die Nährstoffe mit einer Rate von 5 ml/min/Kammer zugeführt.

Nach 24 h wurden die Wundauflagen (2,5 cm x 2,5 cm) vollständig mit simulierter Wundflüssigkeit gesättigt (50 : 50 - "maximum recovery diluent": fötales Kälberserum). Die Wundauflagen wurden anschließend auf die Oberseite der inokulierten Filter aufgelegt, die Abdeckungen der Kammern gesichert und der Reaktor abermals für 24 h in Betrieb genommen.

Nach Ablauf der Zeit wurden die Filter entnommen, in 10 ml "Dey-Engley neutralizing broth" gegeben und für 30 min sonifiziert. Die Proben wurden auf 96-Well Platten verteilt und eine 1:10 Verdünnungsreihe aus jeder Probe mit PBS generiert. Die Verdünnungen wurden auf TSA ausplattiert (20 µl). Nach Inkubation bei 37 °C über Nacht wurden die Kolonien ausgezählt.

Die Ergebnisse der Versuchsauswertung sind als logarithmische Reduktion der Zellzahl der getesteten Organismen in Fig. 7 dargestellt.

### Beispiel 7: Bestimmung der minimalen inhibitorischen Konzentration

Zur Bestimmung der minimalen inhibitorischen Konzentration (MIC) der antimikrobiellen Zusammensetzungen A und B wurden Reihen von Wundauflagen mit jeweils der gleichen antimikrobieller Zusammensetzung aber in unterschiedlichen Beschichtungsmengen getestet. Die Tests fanden gegenüber *P*. *aeruginosa* und S. *aureus* statt. Die Organismen wurden auf eine Konzentration von 10⁵ CFU/mL eingestellt und über einen Zeitraum von 18 h bei 37 °C mit den Wundauflagen in Kontakt gebracht. Die niedrigste Konzentration der jeweiligen antimikrobiellen Zusammensetzung, bei welcher kein visuell erkennbares Erregerwachstum erkennbar war, wird in der nachfolgenden Tabelle als MIC angegeben:

**Tab. 4: minimale inhibitorische Konzentrationen**

| | **MIC [%]** | |
|---|---|---|
| **Zusammensetzung** | ***P. aeruginosa*** | ***S. aureus*** |
| A | 0,002 | 0,0057 |
| B | 0,001 | 0,0048 |

## Patentansprüche

1. Wundauflage umfassend ein Kissen, wobei das Kissen eine erste wundzugewandte Vliesschicht und eine zweite wundabgewandte Vliesschicht aufweist, wobei das Material der ersten Vliesschicht flüssigkeitsdurchlässig ist und sich das Material der zweiten Vliesschicht von dem Material der ersten Vliesschicht unterscheidet, wobei das Kissen weiterhin einen absorbierenden Kern umfassend eine superabsorbierende Substanz enthält und die erste Vliesschicht und die zweite Vliesschicht den absorbierenden Kern einschließen und dabei gemeinsam einen den absorbierenden Kern umgebenden, durchgehenden Randbereich ausbilden, in dem die erste Vliesschicht und die zweite Vliesschicht miteinander verbunden sind,
**dadurch gekennzeichnet, dass** die erste Vliesschicht eine antimikrobielle Zusammensetzung aufweist, die die Inhaltsstoffe
a) ionisches Silber oder Silbernitrat,
b) ionisches Zink, Zinksulfat oder Zinknitrat, und
c) EDTA oder Tetra-Natrium-EDTA
beinhaltet.

2. Wundauflage nach Anspruch 1, **dadurch gekennzeichnet, dass** die Wundauflage weiterhin eine Deckschicht umfasst, die an der zweiten Vliesschicht befestigt ist und ein wasserdampfdurchlässiges und im Wesentlichen flüssigkeitsundurchlässiges Folienmaterial enthält.

3. Wundauflage nach einem der vorhergehenden Ansprüche, **dadurch gekennzeichnet, dass** der absorbierende Kern weiterhin ein absorbierendes Zellstoffmaterial enthält.

4. Wundauflage nach einem der vorhergehenden Ansprüche, **dadurch gekennzeichnet, dass** die erste Vliesschicht zwei verschiedene und zumindest teilweise miteinander verbundene Einzelvliesschichten, nämlich eine innere, wundabgewandte Einzelvliesschicht und eine äußere, wundzugewandte Einzelvliesschicht, umfasst oder aus den zwei verschiedenen Einzelvliesschichten besteht.

5. Wundauflage nach Anspruch 4, **dadurch gekennzeichnet, dass** die innere und äußere Einzelvliesschicht vollflächig miteinander verbunden sind.

6. Wundauflage nach Anspruch 4 oder 5, **dadurch gekennzeichnet, dass** die äußere Einzelvliesschicht aus Polyamidfasern oder Polypropylenfasern besteht und die innere Einzelvliesschicht aus einer Mischung aus Polyamidfasern oder Polypropylenfasern mit Viskosefasern besteht.

7. Wundauflage nach einem der vorhergehenden Ansprüche, **dadurch gekennzeichnet, dass** die erste Vliesschicht insgesamt 50 bis 70 Gew.-% Polypropylenfasern und 30 bis 50 Gew.-% Viskosefasern enthält.

8. Wundauflage nach Anspruch 6 oder 7, **dadurch gekennzeichnet, dass** die Polypropylenfasern oder die Polyamidfasern einer Hydrophilierung unterzogen wurden.

9. Wundauflage nach einem der Ansprüche 4 bis 8, **dadurch gekennzeichnet, dass** die äußere Einzelvliesschicht aus Polypropylenfasern besteht und die antimikrobielle Zusammensetzung trägt und / oder beinhaltet.

10. Wundauflage nach einem der vorhergehenden Ansprüche, **dadurch gekennzeichnet, dass** der absorbierende Kern in ein Zellstoffvlies teilweise oder vollständig eingeschlagen ist, welches sich zwischen dem absorbierenden Kern und der ersten sowie der zweiten Vliesschicht befindet.

11. Wundauflage nach einem der vorhergehenden Ansprüche, **dadurch gekennzeichnet, dass** die antimikrobielle Zusammensetzung die folgenden Mengenverhältnisse enthält:
a) 5 bis 50 Gew.-% ionisches Silber oder Silbernitrat,
b) 2 bis 50 Gew.-% ionisches Zink, Zinknitrat oder Zinksulfat, und
c) 20 bis 93 Gew.-% EDTA oder Tetra-Natrium-EDTA.

12. Wundauflage nach einem der vorhergehenden Ansprüche, **dadurch gekennzeichnet, dass** die antimikrobielle Zusammensetzung 80 bis 92 Gew.-% EDTA, 5 bis 10 Gew.- % Silber und 3 bis 10 Gew.-% Zink enthält.

13. Wundauflage nach einem der vorhergehenden Ansprüche, **dadurch gekennzeichnet, dass** die antimikrobielle Zusammensetzung in Summe 8 bis 10 Gew.-% Silber und Zink enthält.

14. Wundauflage nach einem der vorhergehenden Ansprüche, **dadurch gekennzeichnet, dass** die erste Vliesschicht mit der antimikrobiellen Zusammensetzung beschichtet, imprägniert oder getränkt ist.

15. Wundauflage nach einem der vorhergehenden Ansprüche zusätzlich umfassend eine dritte Vliesschicht, welche die erste Vliesschicht wundseitig überlagert.
